Europäisches Patentamt

European Patent Office

Office européen des brevets

(11) Publication number: **0 504 191 B1**

# EUROPEAN PATENT SPECIFICATION

(45) Date of publication of patent specification: **24.08.94**　(51) Int. Cl.5: **A61K 37/02**

(21) Application number: **91900012.5**

(22) Date of filing: **07.12.90**

(86) International application number:
**PCT/EP90/02127**

(87) International publication number:
**WO 91/08753 (27.06.91 91/14)**

(54) **METHOD AND COMPOSITION FOR THE TREATMENT OF MAMMALIAN HIV INFECTION.**

(30) Priority: **07.12.89 DE 3940526**

(43) Date of publication of application:
**23.09.92 Bulletin 92/39**

(45) Publication of the grant of the patent:
**24.08.94 Bulletin 94/34**

(84) Designated Contracting States:
**AT BE CH DE DK ES FR GB GR IT LI LU NL SE**

(56) References cited:
**US-A- 3 856 936**

(73) Proprietor: **GSF - Forschungszentrum für Umwelt und Gesundheit GmbH**
**Ingolstädter Landstrasse 1**
**Post Oberschleissheim**
**D-85764 Oberschleissheim (DE)**

(72) Inventor: **VOLKER, Erfle**
**Kolberger Str. 7**
**D-8000 München 80 (DE)**
Inventor: **SAERMARK, Torben**
**Gustav Adolfs Torg 43**
**S-211 30 Malmö (SE)**

(74) Representative: **Klunker . Schmitt-Nilson .**
**Hirsch**
**Winzererstrasse 106**
**D-80797 München (DE)**

**Description**

The present invention relates to the use of a compound or composition in the manufacture of a medicament for the treatment of mammalian HIV infections, and more particularly to the use of such a compound or composition for the manufacture of a medicament for treating mammalian HIV infections which comprises hymenoptera venom, or proteinaceous or polypeptide components thereof and which is introduced into the mammalian hosts and which are individually operable to restrict or substantially inhibit the virus replication in the HIV infected cells of the mammal.

The medical profession has long sought an effective method and composition for the treatment of HIV infected individuals. As herein intended, an HIV infection is meant to encompass both HIV1 and HIV2 infections. In this regard, the present research has been directed to seeking a potentially useful therapeutic agent for the manufacture of a medicament for combatting an HIV infection and wherein the composition may inhibit viral reproduction of HIV infected cells, but which does not have numerous deleterious side effects. More particularly, the purpose of such research is to find a composition which is an alternative treatment to the curtent use of AZT. The composition, however, must be operable to selectively destroy HIV infected, or alternatively HIV propagating cells, in an effort to contain or substantially limit the reservoir of available virus cells.

As should be understood, HIV viruses are retroviruses and are therefore most appropriately classified as RNA viruses. The RNA of these particular retroviruses, however, cannot be directly propagated by means of replicase, but rather require an intermediary of DNA synthesis, with the assistance of reverse transcriptase. As should be understood, the DNA serves as a template for the multiplication of the RNA virus. This DNA is later integrated into the host cell genome. This event of integration subsequently leads to the production of new virus cells.

The present invention, which utilizes subtoxic concentrations of hymenoptera venom or proteinaceous or polypeptide components thereof appears to interfere with virus replication at subtoxic concentrations by means of inhibiting reverse transcriptase, and/or selectively inhibiting the growth of HIV infected cells thereby substantially and therapeutically eliminating the virus reservoir with the numerous benefits attendant thereto.

U.S. Patent No. 4,822,608 issued to Benton et al. on April 18, 1989 and entitled "METHODS AND COMPOSITIONS FOR THE TREATMENT OF MAMMALIAN INFECTIONS EMPLOYING MEDICAMENTS COMPRISING HYMENOPTERA VENOM OR PROTEINACEOUS OR POLYPEPTIDE COMPONENTS THEREOF" teaches that secondary agents derived from nature such as hymenoptera venom or proteinaceous or polypeptide components thereof has a potentiating effect on antibacterial agents. This reference further suggests that such compositions may also have increased anti-viral, carcinostatic and anti-carcinogenic effects on various maladies. More particularly, the reference to Benton et al. discloses the use of melittin which is the main component of honey bee toxin, in combination with assorted antibiotic agent as having antibacterial activity against predetermined infections. Further this reference teaches that a synergistic benefit may be achieved by the combination of the melittin and assorted antibiotics in various therapeutically effective amounts.

As discussed in detail in the prior art reference to Benton et al., melittin, the main component in honey bee toxin is a polypeptide which includes substantially 26 amino acid residues. These amino acid residues include,

```
                                                        Gly-Ile-
Gly-Ala-Val-Leu-Lys-Val-Leu-Thr-Thr-Gly-Leu-Pro-Ala-Leu-Ile-
Ser-Trp-Ile-Lys-Arg-Lys-Arg-Gln-Gln-Amide.
```

Moreover, the inventors have discovered a direct effect of melittin analogues and wherein at least the last six (C-terminal) amino acids are altered and replaced by six glycine residues appear to have a therapeutic benefit similar to melittin, these amino acid analogues having a structure of

Gly-Ile-Gly-Ala-Val-Leu-Lys-Val-Leu-Thr-Thr-
Gly-Leu-Pro-Ala-Leu-Ile-Ser-Trp-Ile-Gly-Gly-Gly-Gly-Gly-Gly.

It is furthermore known from the Merck Index (1983) 10th Ed. no. 5643 that melittin can be used as an anti-rheumatic agent.

US-A-3,856,936 discloses a composition for cortisol level control in mammals by topical application consisting of an effective amount of a member selected from the group consisting of a whole bee venom and melittin in a mixture with cocoa butter fatty acids.

Therefore, several medical uses of hymeoptera venom or proteinaceous or polypeptide components thereof or melittin are already known. There is, however, no mention in the known literature for the use of melittin or hymenoptera venom in the manufacture of a medicament for the treatment of HIV infections.

It is therefore an object of the present invention to provide a compound or composition for use in the manufacture of a medicament for the treatment of HIV infections in mammals and which comprises hymenoptera venom, or proteinaceous, or polypeptide components thereof.

Another object of the present invention is to provide such a compound or composition for use in the manufacture of a medicament for the treatment of HIV infections in mammals wherein the hymenoptera venom consists essentially of honey bee venom, bumble bee venom, yellow jacket venom, baldface hornet venom, active protein components of said venom, active polypeptide components of said venom and/or mixtures thereof.

Another object of the present invention is to provide an effective subtoxic dosage of a structural analogue of melittin or melittin itself for use in the manufacture of a medicament for the treatment of HIV infections in mammals whereby virus replication in the HIV infected cells of the mammal is substantially inhibited.

Another object of the present invention is to provide an effective, subtoxic dosage of a polypeptide mixture of melittin and structural analogues thereof for use in the manufacture of a medicament for the treatment of HIV infections in mammals whereby replication of viral cells in the HIV infected cells are inhibited.

Another object of the present invention is to provide Amfi 1, Amfi 2 and peptides of GP 41 that are melittin-like for use in the manufacture of a medicament for the treatment of an HIV infection in a mammal.

Further objects and advantages of the present invention are to provide a compound or composition for use in the manufacture of a medicament for the treatment of mammalian HIV infection which is safe and effective, and which further avoids the detriments individually associated with the prior art therapy for this same malady.

Fig. 1 is a graphic illustration which shows a comparison of cell growth achieved with non-infected and HIV infected cells as a percentage of untreated controls and which are interdependent with the melittin concentration.

Fig. 2 is a graphic illustration which shows the activity of reverse transcriptase (RT%) as compared with a standardized cell line, and as further compared with the infectivity (INF%) of the culture supernatant of HIV infected T-lymphoma cells (KE37-1/111$\beta$) as a percentage of untreated controls and which are interdependent with the concentration of melittin.

Fig. 3 is a graphic illustration which shows a comparison of the cell growth achieved for non-infected and HIV infected cells as a percentage of untreated controls and which are interdependent with the concentration of melittin-analogues.

Fig. 4 is a graphic illustration of the activity of reverse transcriptase (RT%) as compared with a standardized number of cells and the infectivity (INF%) of the culture supernatant of HIV-infected T-lymphoma cells (KE37-1/111$\beta$) as a percentage of untreated controls and which are interdependent with the concentration of melittin analogues.

Fig. 5 is a graphic model of the carboxyterminal region of the GP41 molecule of the HIV virus.

Fig. 6 is a graphic illustration which shows a comparison of cell growth achieved for HIV infected, and non-infected cells as a percentage of untreated controls and which are interdependent with the concentration of melittin.

Fig. 7 is a graphic illustration which shows the percentage of infectious cells as a function of the concentration of melittin.

Fig. 8 is a graphic illustration which shows a comparison of cell growth achieved with HIV infected and non-infected cells as a percentage of untreated controls and which are interdependent with the concentration of melittin 6.

Fig. 9 is a graphic illustration which shows the percentage of infectious cells as a function of the concentration of melittin 6.

Fig. 10 is a graphic illustration which shows the percentage of cell growth achieved for HIV infected and non-infected cells as a percentage of untreated controls and which are interdependent with the concentration of melittin 4.

EP 0 504 191 B1

Fig. 11 is a graphic illustration which shows the percentage of infectious cells as function of the concentration of melittin 4.

Fig. 12 is a graphic illustration which shows the percentage of cell growth achieved for HIV infected and non-infected cells as a percentage of untreated controls and which are interdependent with the concentration of melittin E.

Fig. 13 is a graphic illustration which shows the percentage of infectious cells as a function of the concentration of melittin E.

Fig. 14 is a graphic illustration of the percentage of cell growth achieved for HIV infected and non-infected cells as a percentage of untreated controls and which are interdependent with the concentration of melittin F.

Fig. 15 is a graphic illustration which shows the percentage of infectious cells as a function of the concentration of melittin F.

Fig. 16 is a graphic illustration which shows the percentage of cell growth achieved for HIV infected, and non-infected cells as a percentage of untreated controls and which are interdependent with the concentration of melittin 3.

Fig. 17 is a graphic illustration which shows the percentage of infectious cells as a function of the concentration of melittin 3.

Fig. 18 is a graphic illustration which shows the percentage of cell growth achieved for HIV infected and non-infected cells as a percentage of untreated controls and which are interdependent with the concentration of melittin 1-20.

Fig. 19 is a graphic illustration which shows the percentage of infectious cells as a function of the concentration of melittin 1-20.

Fig. 20 is a graphic illustration which shows the percentage of cell growth achieved for HIV infected and non-infected cells as a percentage of untreated controls and which are interdependent with the concentration of mastoparan.

Fig. 21 is a graphic illustration which shows the percentage of infectious cells as a function of the concentration of mastoparan.

Fig. 22 is a graphic illustration which shows the percentage of cell growth achieved for HIV infected and non-infected cells as a percentage of untreated controls and which are compared with the concentration with AMFI 2. The percentage of cell growth for HIV infected and non-infected cells as a percentage of untreated controls and which are compared with the concentration of AMFI 1 is identical to the results depicted in Fig. 22.

Fig. 23 is a graphic illustration which shows the percentage of infectious cells as a function of the concentration of AMFI 2. The percentage of infectious cells as a function of the concentration of AMFI 1 is identical to the results depicted in Fig. 23.

Fig. 24 is a graphic illustration which shows the percentage achieved for cell growth of HIV infected and non-infected cells as a percentage of untreated controls and which are compared with the concentration of MHC peptide.

Fig. 25 is a graphic illustration which shows the percentage of infectious cells as a function of the concentration of MHC peptide.

Fig. 26 is a graphic illustration which shows the percentage achieved for cell growth of HIV infected and non-infected cells as a percentage of untreated controls and which are compared to the concentration of DMSO (solvent control).

Fig. 27 is a graphic illustration which shows the percentage of infectious cells as a function of the concentration of DMSO (solvent control).

Fig. 28 is a graphic illustration which shows the percentage of cell growth achieved for HIV infected and non-infected cells as a percentage of untreated controls and which are compared with the concentration of the HOLST peptide (negative control).

Fig. 29 is a graphic illustration which shows the percentage of infectious cells as a function of the concentration of the HOLST peptide.

Fig. 30 is a graphic illustration which shows the percentage of P24 production as compared with the concentration of melittin at various time intervals.

Fig. 31 is a graphic illustration which shows the percentage of P24 production in cells and supernatant in comparison to the concentration of melittin at a period of three hours.

Fig. 32 and 33 are graphic illustrations which shows the concentration of P24 in cells and supernatants following three hours and fourteen hours of incubation in the presence of melittin respectively.

Fig. 34 is a graphic illustration which shows the influence of melittin on the P24 determination in cell free HIV supernatants at three and fourteen hours, respectively, after incubation with various concentrations

4

of same.

Fig. 35 is a graphic illustration which shows a prolonged toxicity study with mice, and more particularly the effective body weight of the mice as it relates to the various venoms which were used during the toxicity study and which were taken at various times after the onset of dosing with the particular venom.

Fig. 36 is a graphic illustration which shows the body weight of the mice in the prolonged toxicity study as a function of the number of days after the onset of dosing with the substance used in the toxicity study.

Fig. 37 is a graphic illustration which shows the body weight of individual mice as a function of the use of honey bee venom at various days after the onset of dosing.

Fig. 38 is a graphic illustration which shows the body weight of the mice as a function of the use of yellow jacket venom at various days after the onset of dosing.

Fig. 39 is a graphic illustration which shows the body weight of the mice as a function of the use of hornet mixture at various days after the onset of dosing.

Fig. 40 is a graphic illustration which shows the body weight of mice as a function of the use of a vespid mixture at various days after the onset of dosing.

Fig. 41 is a graphic illustration which shows a prolonged toxicity study for mice and which summarizes the microscopic examination of the various anatomical parts of the respective mice receiving the honey bee and yellow jacket mixture following the toxicity study.

Fig. 41A provides a legend for the various data presented in Fig. 40.

Fig. 42 is a graphic illustration of the structure of melittin and the structural analogues thereof.

The KE37-1 (uninfected) and KE37-1/111$\beta$ (infected) cell lines discussed below are commercially available from the Dr. Robert Gallows Laboratory of the NIH in the United States of America and grown in a medium of RPMI 1640 (GIBCO) and further supplemented with 10% fetal calf serum which contained approximately 100 units of penicillin, 100$\mu$g streptomycin, and approximately 0.25$\mu$g fungizone (Amphotericin B) per milliliter. The human mesenchymal cell lines LC5 and LC5-HIV, discussed below, are deposited with the Collection De L'Institut Pasteur of Paris, France on March 9, 1989 and given the accession numbers I-842 and I-843, respectively. The LC5 and LC5-HIV cell lines were grown in a medium of RPMI 1640 and supplemented with 10% fetal calf serum.

The melittin peptides and analogues from GP41 were synthesized using a commercially available peptide synthesizer (model Biolynx, Pharmacia Biochrome, Cambridge UK) and the preveighted amino acid OPFP esters supplied for this machine (Pharmacia Biochrome, Cambridge, UK). The synthesis strategy was based on the Fmoc strategy as described in the manual for the equipment. The acylation rate was monitored by the Bioplus software using the release of anionic dye (Acid violet 17, 30 mg pr 100 ml dimethylformamide and 0,14ml diisopropylethylamine) at 600nm according to the protocol supplied by the manufacturer. The principle is known as counter ion distribution monitoring, CDM, and is described by Salisbury, S.A., Treemeer, E.J., Davies, J.W. and Owen, D.,E.,I.,A., (1990) J.Chem.Soc., Chem.Commun., 1990, p. 538-540. The linkers used resulted in release of peptide amide (Ultrosyn C, Pharmacia Biochrome, Cambridge, UK).

The first coupling to the acid labile linker Ultrosyn C (0.1meq) was carried out using a symmetrical anhydride (0.4 mcq) and addition of dimethylaminopyridine (0.05 meq). This resulted in at least 80% coupling after 1 hour as determined by release of the Fmoc group and untreated sites were capped using acetic anhydride. The subsequent couplings were carried out using commercially available active esters (Pharmacia Biochrome, UK) with a coupling time determined by the CDM carried out automatically by the software used (above). Typical coupling times were typically 1 hour using 4 times excess of the esters. Ser was coupled using dihydroxybenzotriazole esters until colourless (1.5 - 2 h). The Fmoc group was removed by 5 bed volumes of piperidine (20% in dimethylformamide). The dimethylformamide was distilled before use and essentially amine free as determined by the dinitrofluorobenzene test as described by the protocol for the Biolynx. None of the couplings offered particular problems and the crude peptides appeared more than 80% pure as determined by high pressure liquid chromatography (HPLC, see below).

The peptides were cleaved from the resin using trifluoroacetic acid with the addition of 2% anisole and 2% ethanedithiol for 2 hours followed by ether precipitation. The peptide was purified to more than 95% purity by HPLC on a TSK 120T reverse phase column (7.5 x 300 mm) (Pharmacia, Sweden). The peptides typically eluded at 65% acetonitrile (between 55 and 75%) using a linear gradient over 90 minutes from 0 to 80% acetonitrile in 0.1% trifluoroacetic acid. The sequence was verified by protein sequencing on an Applied Biosystem sequencer according to the manufacturer.

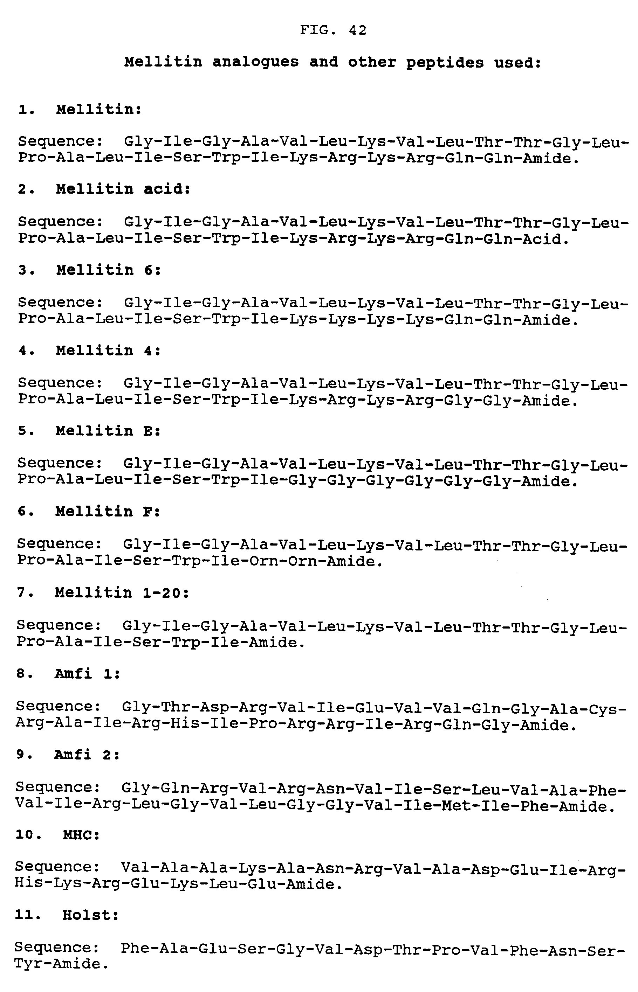

The synthesis of GP41 analogues and mastoparan were carried out as described for the melittins. A listing of the melittin analogues and other peptides used is depicted in Fig. 42.

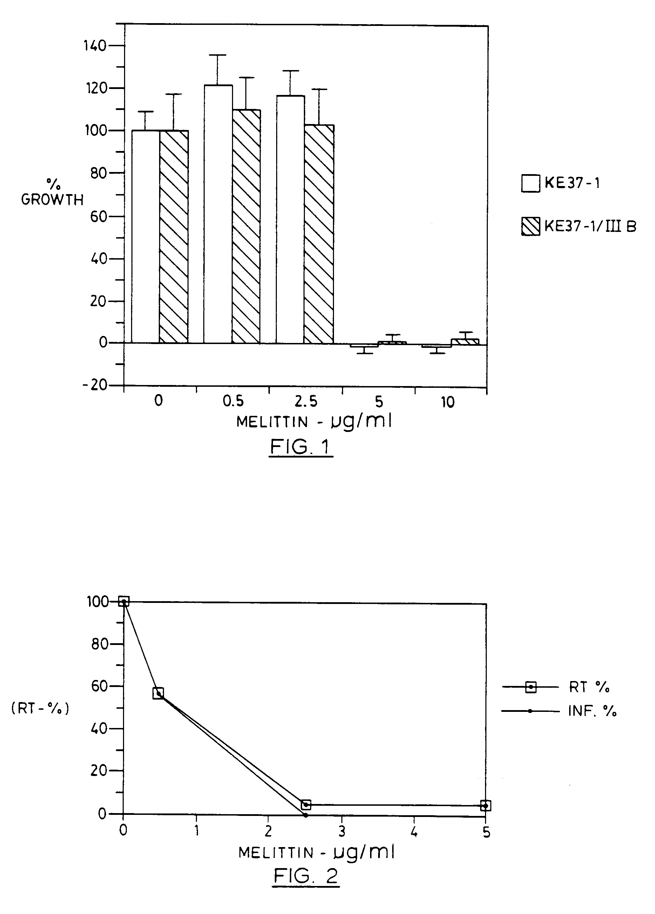

Referring more particularly to Figs. 1-4, melittin and its specified analogues were tested for their effect on HIV-infected T-lymphoma cells. In this regard the following cell lines were employed in the testing:

5

KE37-1 (uninfected) and KE37-1/111β (infected with HTLV-111β). These infected cells were incubated for a period of seven days under test conditions at a temperature of 37°C and in the presence of 5% $CO_2$. A culture medium of RPMI 1640 (GIBCO) was employed and was further supplemented with 10% fetal calf serum which contained approximately 100 units of penicillin, 100μg of streptomycin, and approximately 0.25μg fungizone (Amphotericin B) per milliliter. This equals 1 milliliter of antibiotic-antimycotic solution (GIBCO Catalogue No. 043-05240 per 100 ml. of medium.) After a one week incubation period in the presence of various concentrations of the above-identified substances, which were added fresh daily to the medium, the following tests were carried out with the cultures. A first test was conducted for purposes of determining the relative cell concentration as a function of the number of HIV infected cells present by employing an MTT test. The MTT test was carried out in microtiter plates according to T. Mosmann, "Rapid Colorimetric Assay for Cellular Growth and Survival: Application to Proliferation and Cytotoxicity Assays," Journal of Immunological Methods, v. 65 (1983), pp. 55-63. The MTT test was slightly modified: 10μl of an MTT solution (5 mg/ml MTT dissolved in PBS, sterlized by filtration; MTT is 3-(4,5-Dimethylthiazole-2-yl)-2,5-diphenyltetrazolimbromide) were added to every well. The cells were incubated with MTT for four hours at 37° in 5% $CO_2$ atmosphere. The yellow MTT is reduced to a blue formazane by metabolically active cells. The reaction was stopped by adding 200μl of 0.04 N HCl in isopropanol to every well and the dye was extracted. The resulting solutions were vigorously mixed in order to dissolve all the formazene crystals. The optical density (OD) of the solution was determined at = 600 nm. The optical density is proportional to the cell density under these conditions.

The second test was conducted to determine the activity of reverse transcriptase in the supernatant of treated, HIV infected cell culture, by employing the test described by Poiesz et al. and which is found at PNAS 77 (1980): 1415-1419 and which was subsequently modified. A third test was conducted for purposes of determining the relative infectious nature of the supernatant of treated, HIV infected cell cultures. In this regard, uninfected, HIV receptive human embryonal lung cells (LC5) were incubated in the culture supernatant which was to be tested, and this was subsequently followed by a three day cultivation of the same cells in fresh media. These cells were then tested for the production of HIV specific proteins.

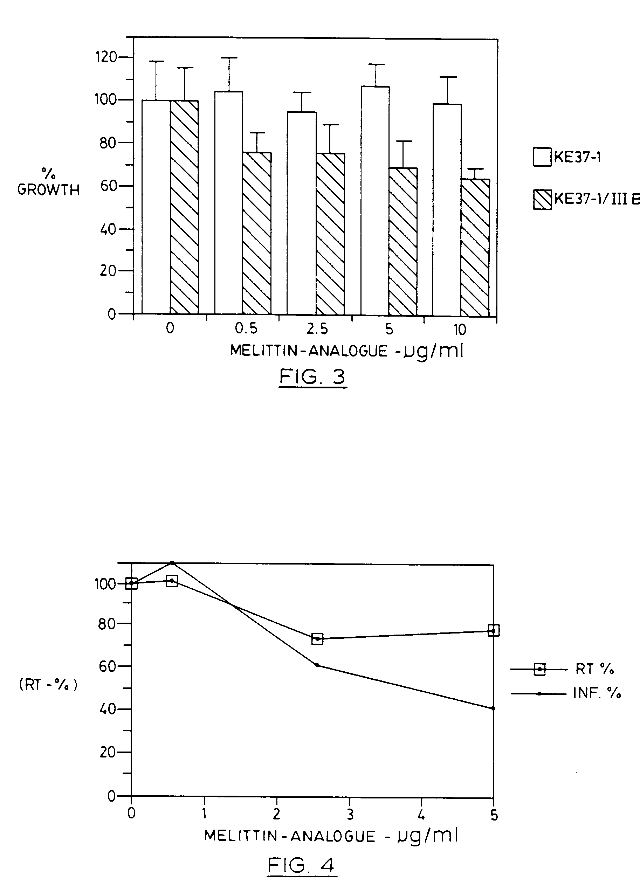

The process of verifying the presence of HIV proteins was achieved through serological testing wherein antibodies to these proteins, that is primary antibodies and antibodies, directed to corresponding immunoglobulins, that is, secondary antibodies, and to which horse radish peroxidase is coupled, are employed. To visualize the antibody-antigen complex 3-aminoethylcarbazol was employed. This substance is not soluble in water. This procedure is better known as indirect immunoperoxidase coloration. This procedure is described at Mellert et al., "HTLV-III/LAV - Antikorpertest: Indirekte Immunperoxidasefärbung (HTLV-III/LAV antibody test: Indirect Immunoperoxidase staining," AIDS-FORSCHUNG (AIFO) February 1986 Heft 2, pp. 105-107. As best seen in Figs. 1-4, it should be noted that melittin is toxic to both HIV infected cells as well as non-infected cells at concentrations above 5μg per ml. In this regard, toxic studies relative to mice are provided hereinafter to further aid in analysis of the present test data. In addition to the foregoing, the specified melittin analogue as shown in Fig. 3 appears clearly less toxic than melittin. However, this same analogue develops a selective growth inhibiting effect with respect to HIV infected cells at higher dosages, that is, 10μg per ml. Further, it should be noted that with a concentration of 5μg per ml., at which melittin is toxic to both infected, as well as non-infected cells, the inventors have found that the growth of infected cells are reduced by almost 80% by utilizing melittin analogues after a period of approximately seven days while the growth of non-infected cells does not appear readily affected.

In addition to the foregoing, the test data as shown in Figs. 1 through 4 illustrates that with a melittin concentration of 2.5μg per ml., at which the level of growth of the cells appears not yet influenced, the ability of the cells to infect, as well as the activity of reverse transcriptase appear to revert substantially back to zero. This is shown most clearly by reference to Fig. 2.

The test data, as best seen by reference to Fig. 4, also shows that melittin analogues are also capable of lowering the activity of reverse transcriptase and the quantity of infectious virus cells in supernatant-treated HIV infected cells. This effect appears to occur in concentration ranges which are not yet toxic for non-infected cells, but clearly toxic for HIV-infected cells.

In summary, the test data shown in Figs. 1-4 shows that melittin appears therapeutically useful for inhibiting viral replication at subtoxic concentrations by means of the inhibition of reverse transcriptase. Furthermore, melittin appears to inhibit the growth of HIV infected cells selectively which makes the elimination of the virus reservoir possible in mammals.

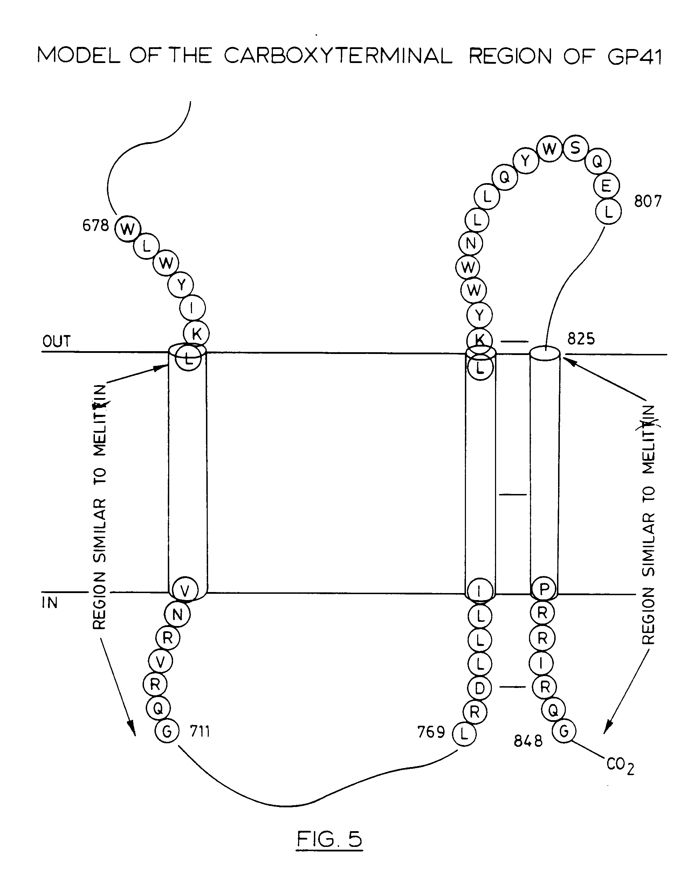

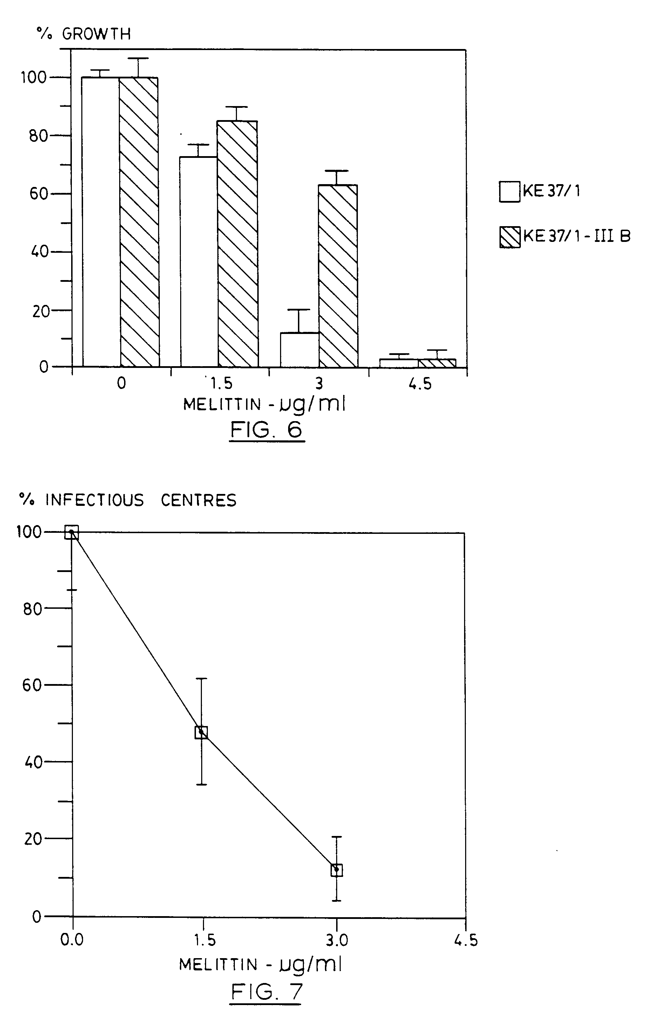

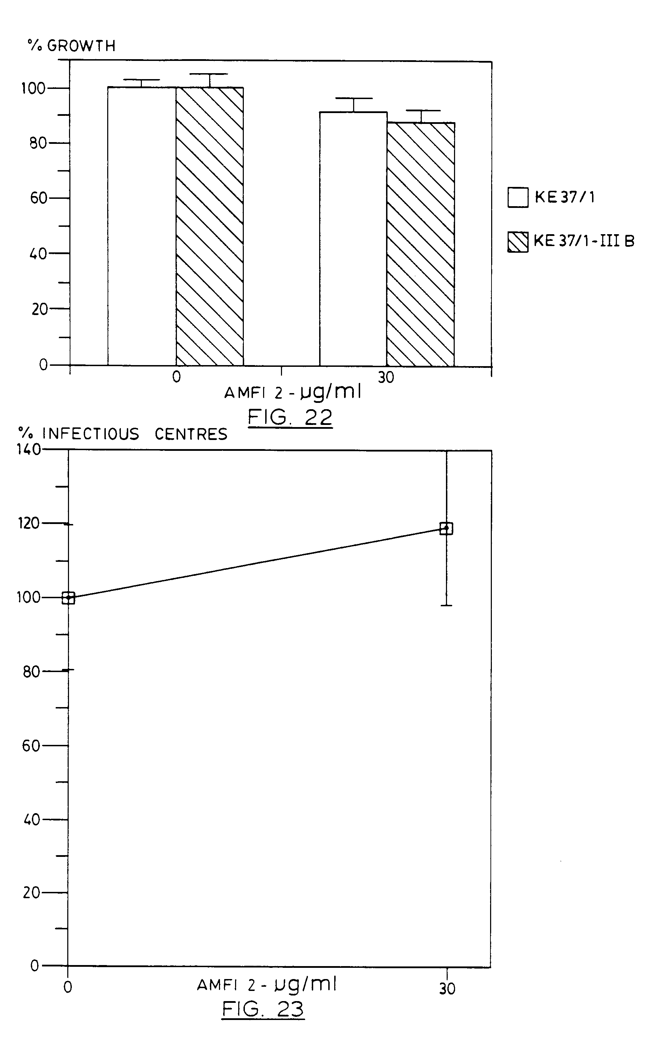

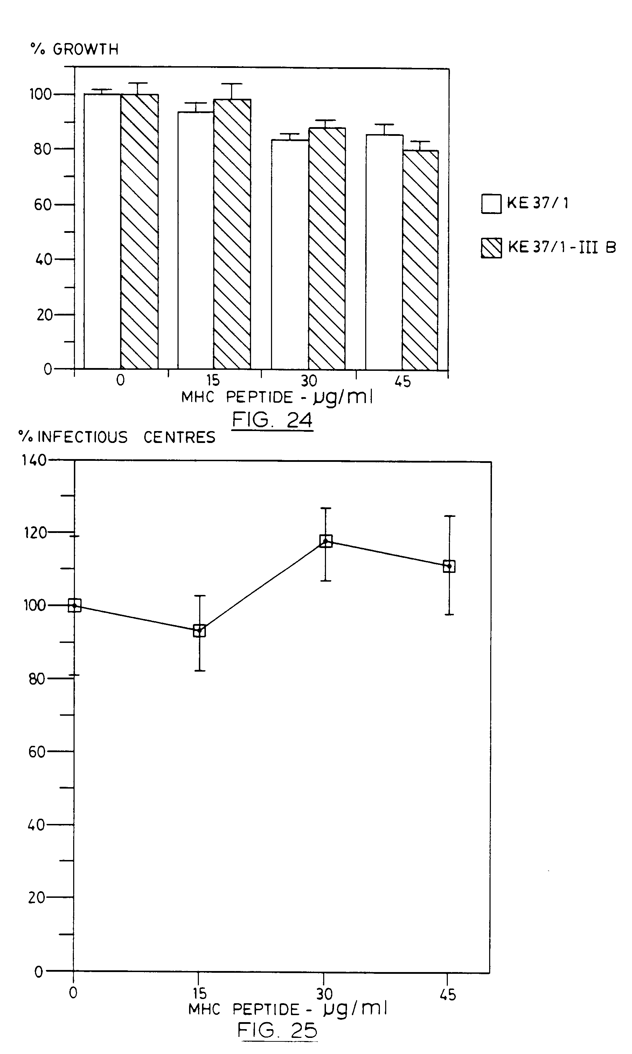

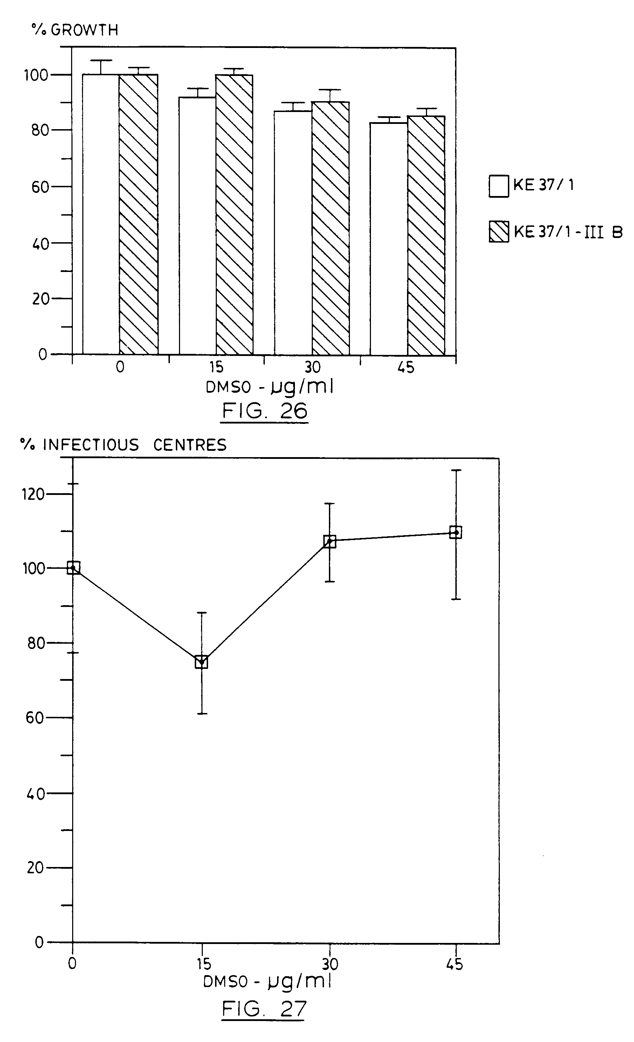

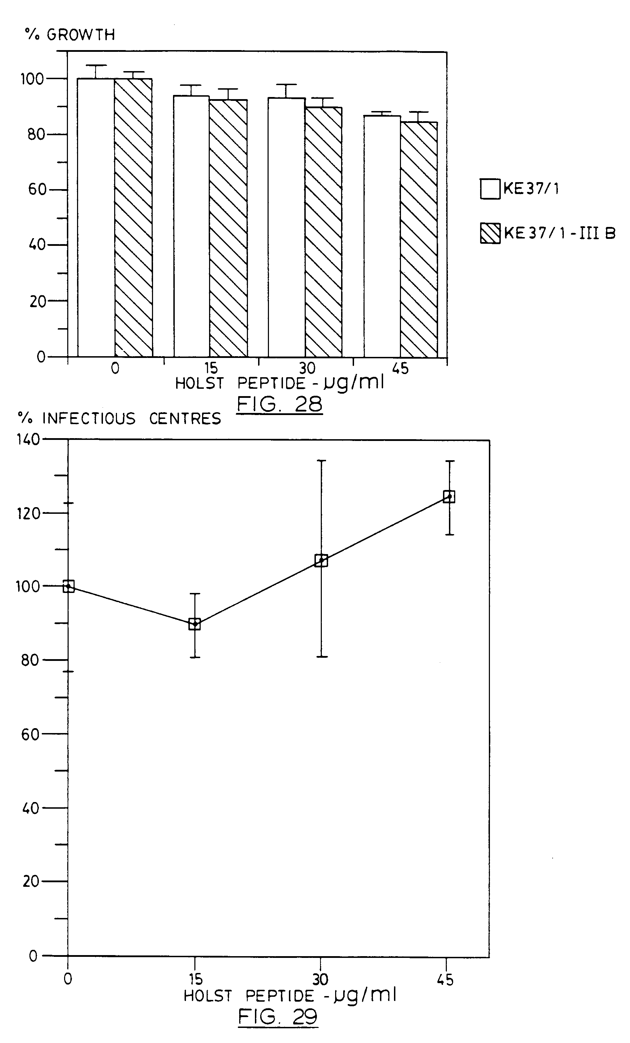

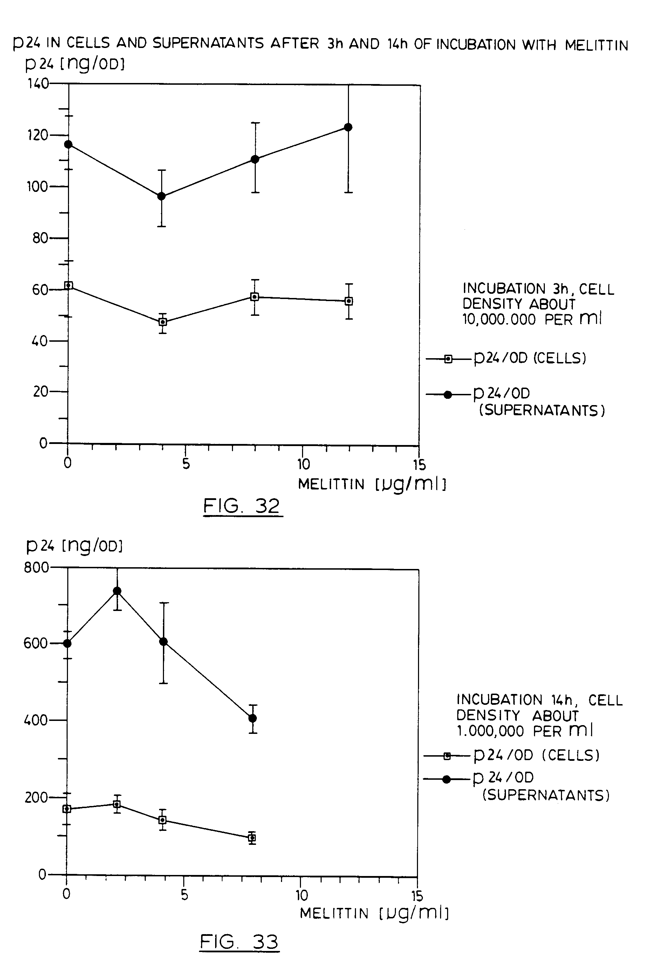

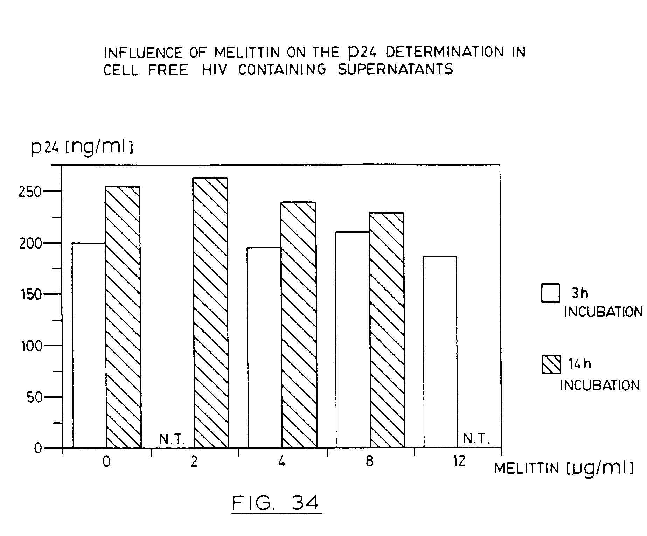

Additional tests were conducted to further supplement the test results shown in Figs. 1 through 4. In these tests, which are illustrated most clearly by reference to Figs. 6 through 34, respectively, the possible mechanism(s) by which melittin inhibits the growth of HIV infected cells was investigated. By way of introduction and referring more particularly to Fig. 5, energy minimization studies of the surface of HIV protein GP41 was conducted using the CHARMM algorithm on secondary structure predictions which were based on neural network computing principles. As should be understood, the application of a neural network program as used herein is to predict secondary protein structures from amino acid sequences. This network has been trained to classify amino acid residues into three types of secondary structures: alphahelix, beta-sheet and random coil. By training it on a large set of proteins with known secondary structure, the neural network becomes able to make predictions about the secondary structure of proteins novel to the network, purely on the basis of their primary structure. An analysis of the secondary structure of the human immunodeficiency virus (HIV protein GP 41) by computer modeling based on neural network methods may be found at H. Andreassen et al., "Analysis of the Secondary Structure of the Human Immunodeficiency Virus (HIV) Proteins p17, gp120, and gp41 by Computer Modeling Based on Neutral Network Methods," Journal of Acquired Immune Deficiency Syndromes, v. 3 (1990), pp. 615-622. More particularly, the conclusions drawn from these studies shows that two transmembrane sections of the GP41 protein have a certain homology to the structure of melittin. In this regard, this homology is quite noteworthy inasmuch as other analogues of melittin which are quite different from melittin appear to have the same HIV inhibiting effect. It appears, therefore, that several of the amino acids in melittin can be replaced without changing the activity of the molecule provided that the amphiphilicity is conserved, and polymerization of four melittin molecules by charge interaction is possible. The conclusions drawn from these tests, and which will be discussed in further detail hereinafter, is that melittin appears to interact with one of the major HIV proteins, glycoprotein GP41. This appears possible because of the similarity between the transmembrane regions of GP41 and melittin as shown in Fig. 5.

As should be understood, melittin is an amphiphilic peptide which is 26 amino acids long. In an aqueous solution of high ionic strength, melittin adopts a tetrameric configuration. At low ionic strengths, however melittin assumes a monomeric random coil. At physiologic ionic strengths the distribution between these two forms appears to be equal. Previous studies have shown that melittin appears to be able to adapt to the hydrophobic environment of a cell membrane by forming a monomeric alphahelix with the electrical charges distributed on one side of the helix and the uncharged side associated with the membrane. This research further indicates that the surface of the melittin polymer facing the surrounding lipid is thus uncharged. Further, another peculiar feature of the melittin molecule is that a kink of approximately 120° is introduced into the melittin helix by a proline (amino acid No. 14). A similar kink is found in the HIV GP41 melittin-like sequence which was discussed above.

Earlier studies have shown that melittin is known to have several effects. These effects are best described as a cell surface binding process and a polymerization process wherein alphahelices align to form a channel. Further, it has been shown that binding by the C-terminal, that is the 6 amino acids of the tail appears to be necessary for cell lysis. This fact is very important for understanding the experimental test data which will be described hereinafter. As should be understood the precise mechanism by which melittin forms a channel is not understood. It appears, however, that the melittin molecule binds to the phosphate anions with its basic C-terminus, thereby interacting with ten (10) phosphatidylcholine molecules. Once the melittin molecule is anchored, it appears to turn into the outer lipid layer of the membrane thereby disturbing the structure of the membrane. This activity is followed by formation of pores through the outer layer of the membrane thereby increasing ion permeability and a subsequent rupture of the membrane. The channels formed in the membrane appear to be stabilized by the melittin molecules. The formation of the channel also leads to lysis of the cells and which will hereinafter be referred to as the toxicity of melittin. This can be compared to the mechanism by which alphatoxin and complement are cytotoxic. Although these molecules may be capable of spanning the membrane thereby forming a pore with the same attendant results.

In addition to the effects involving the channel-forming possibilities of amplilic peptides; an effect on phospholipase A2 has been described and which may also involve the amphiphilic interaction between two proteins. In this case, melittin may perhaps interact with the hydrophobic parts of the enzymatic protein phospholipase A2 or it may be due to melittin-phospholipid interactions. In addition to the foregoing, it is possible that the effects of melittin may also be intracellular. It is known, for example, that melittin stimulates endogenous phospholipase A2 in the anterior pituitary leading to growth hormone secretion.

A series of experiments and which are illustrated graphically in Figs. 6 through 34, respectively, were designed to detect two extremes. In particular the tests were designed to detect the inhibition of viral release and/or the release of virus which has a reduced ability to infect other cells. This reduced ability to infect other cells is represented, for example, by glycosylation inhibitors, the direct effect on virus release is represented by acylation inhibitors, RT inhibitors and melittin. The most important feature of this experimental design, however, is to detect the toxicity of melittin. The present experiments were carried out on both the astrocytoma cell line LC5-HIV (cloned infected cells) and T-lymphocytoma cell line KE37-1/III$\beta$ (cloned

infected cells with HTLV-III$\beta$). In both cases the results were essentially identical.

In the present series of experiments HIV release was tested by a fully automated laboratory robot system and which is manufactured by Beckman Instruments as the Biomek 1000. This system performed the following steps. First, chronically infected cells (cloned) are plated in microtiter plates and grown for a period of seven days. They are then challenged by melittin during this period either for the final day or throughout the period as shown in the figures. The number of living cells are quantified by MTT, that is by a metabolic (dehydrogenase) test. The MTT test is automatically performed. The toxic effect is detected at the time of the experiment and not in a separate control experiment. Furthermore, the supernatant, and which contains released virus cells from the period of time which elapsed from the last wash, and which may vary from 24 hours to 7 days, is collected from the culture, and an aliquot is saved for virus quantification by employing antigen or viral enzyme reverse transcriptase (RT). Furthermore, an aliquot is added to a culture of uninfected cells in microtiter plates for subsequent measurement of the ability of the virus cells to reinfect these uninfected cells. In this regard, the number of infected cells is automatically quantified by an immunoperoxidase staining technique. The test results, as shown, are expressed in terms of toxicity of the melittin (MTT test); the release of HIV from the primary culture (RT test); and the ability of the released virus to infect previously uninfected cells.

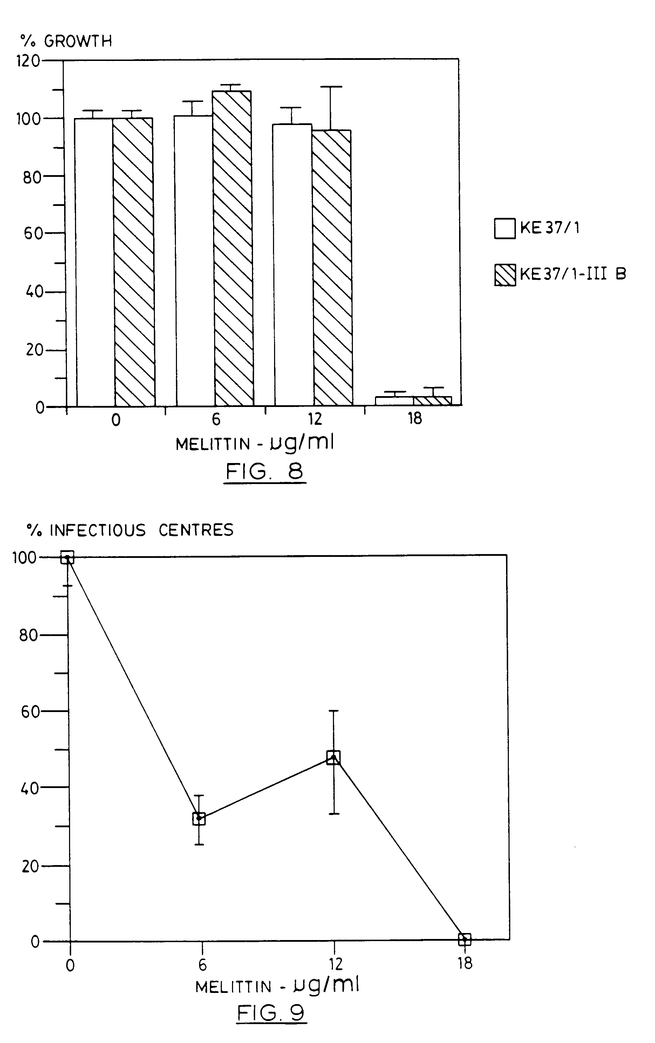

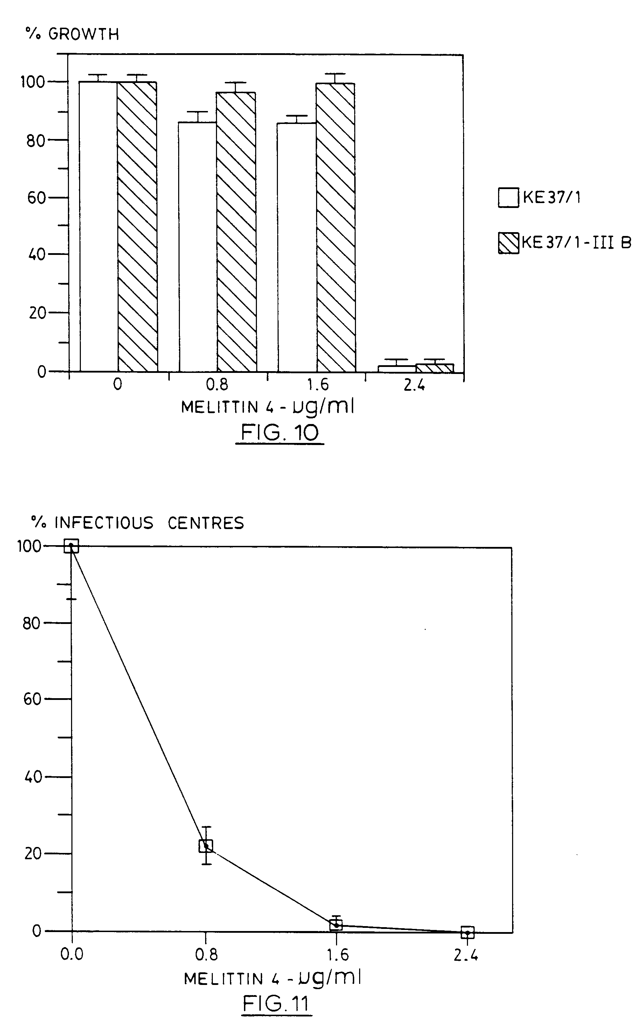

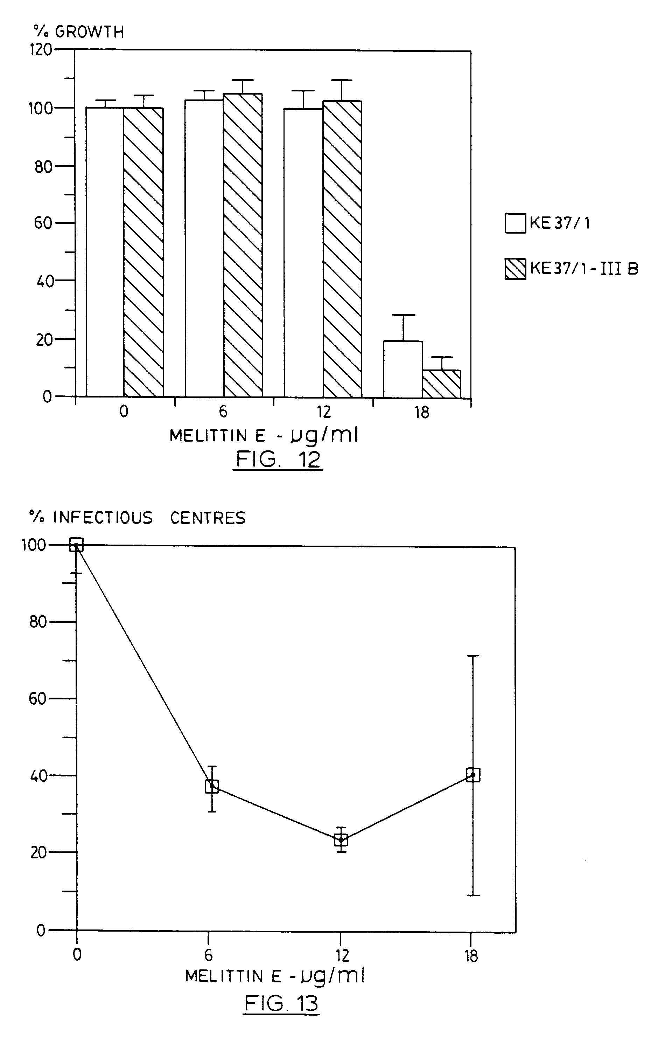

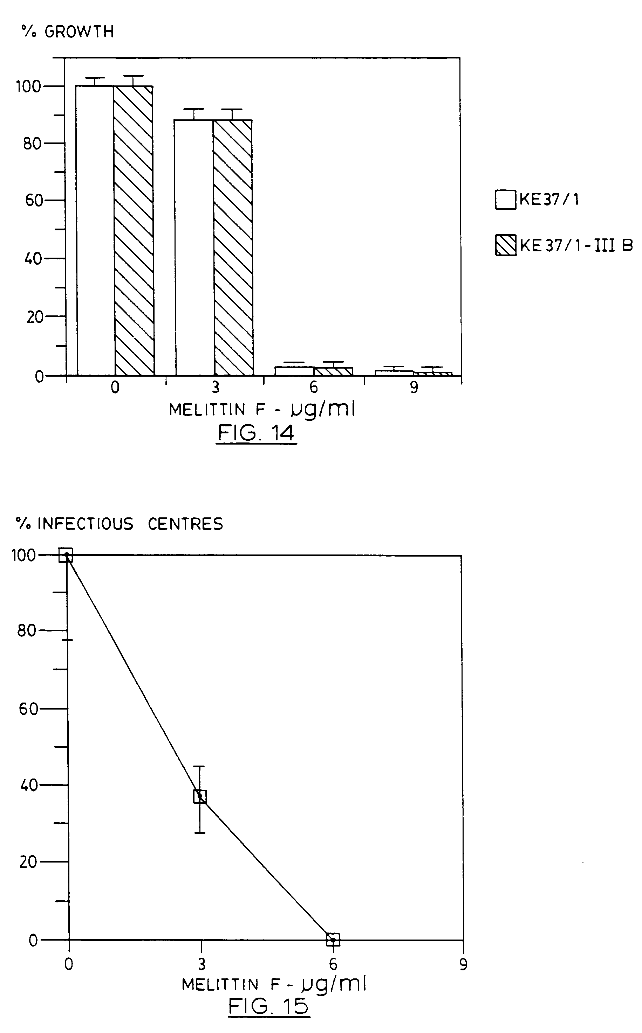

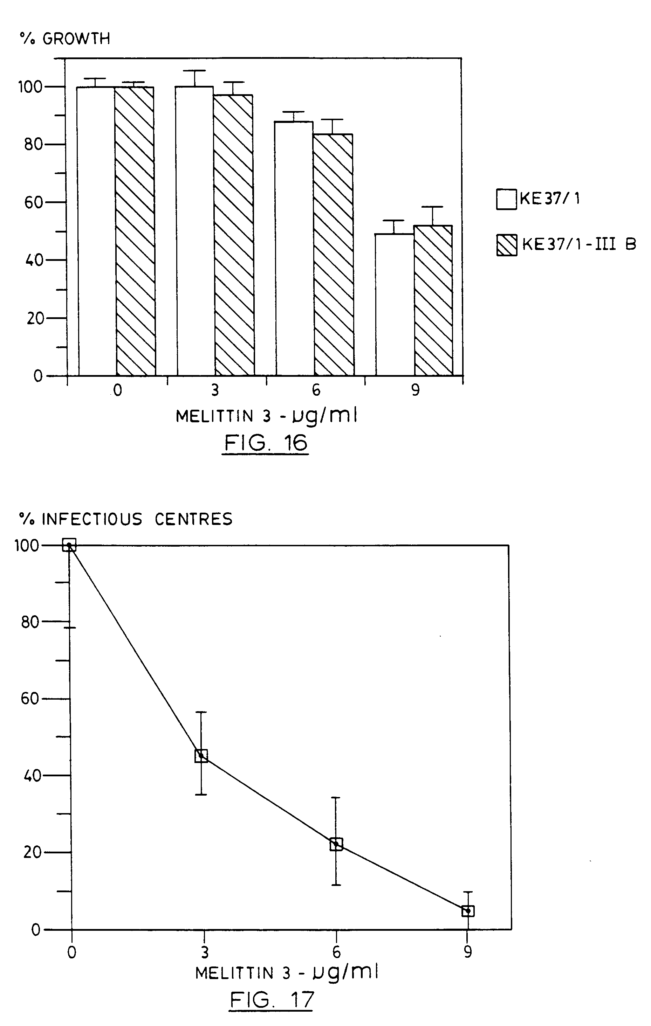

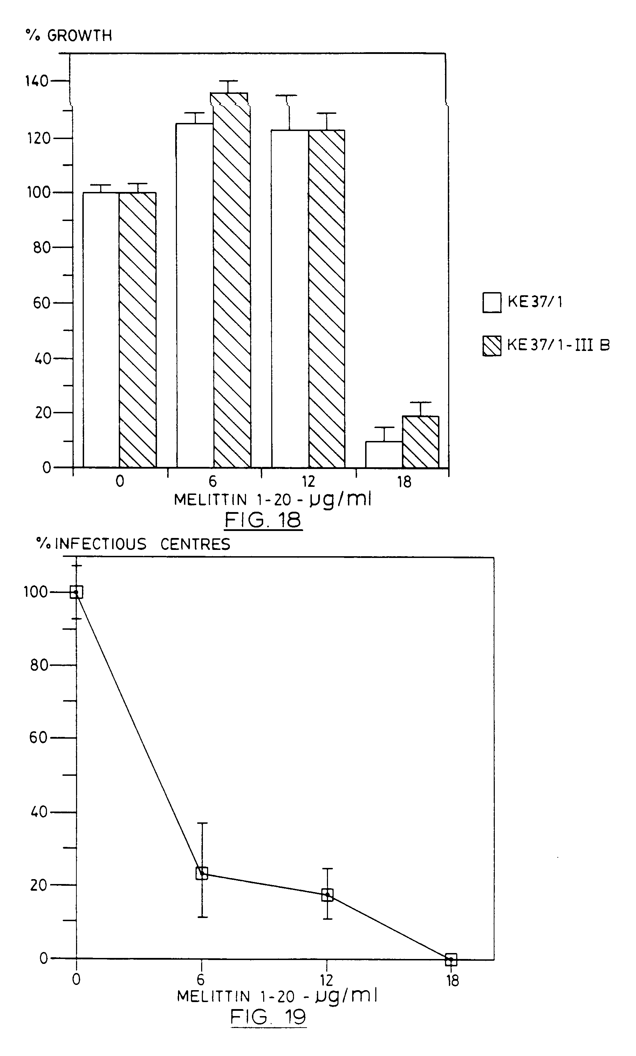

As best seen by reference to Figs. 6 and 7 the overall effect of melittin on HIV infected cells appears to be the inhibition of viral cell release from infected cells (Fig. 6), but in its natural form it appears to not selectively affect the infected cells (Fig. 7). This relationship is also shown with respect to melittin 6 (Figs. 8 and 9); melittin 4 (Figs. 10 and 11); melittin E (Figs. 12 and 13); melittin F (Figs. 14 and 15); melittin 3 (Figs. 16 and 17); and melittin 1-20 (Figs. 18 and 19); respectively. The information derived from this test data is noteworthy inasmuch as the membranolytic effect of melittin appears dependent on the surface binding of the melittin to cells via its C-terminal. As earlier discussed, this binding process also appears dependent on positive charges due to the C-terminal sequence which includes an amino acid sequence of Lys-Arg-Lys-Arg-Gln-Gln-Amide. To determine whether the effect of melittin was based on the known mechanism of channel formation or due to an unknown process the inventors tested (1-20)-6-(Gly)-Amide. It was reasoned that due to its lack of charges this form of melittin should act in a fashion consistent with its amphiphilic properties and not be dependent on the specific interaction with the cell surface. Contrary to the inventors' hypothesis, however, and as best seen by reference to Figs. 18 and 19, it appeared clear that melittin analogues that do not specifically bind to the cell surface and are therefore much less toxic appear to inhibit viral cell release and also selectively kill HIV infected cells. This experimental data tends to indicate that the toxic, cytolytic effective of melittin may conceivably mask the selective killing of infected cells. This would be expected if the lytic properties of melittin were not involved in its anti-HIV effects. It is thus a novel and hitherto unexplored aspect of melittin action.

The test results further clearly shows that natural melittin was able to lyse HIV-infected LC5 cells. The test data showed, for example, that the nature of this action appeared to be related to the critical concentration phenomenon of same. More particularly, and when the critical concentration was reached (10$\mu$g per ml.) all cells, that is, infected and non-infected, were killed. This effect happened at concentrations ten times higher than the effect on HIV release. Melittin COOH was tested and appeared to be less efficient than the melittin amid. In addition to the foregoing, two melittin analogues were tested, which lacked the binding tail of positively charged amino acids in positions 21 through 26. These melittin analogues both had the same pronounced effect on HIV infected cells, that is these infected cells were selectively killed. For example, and at 10$\mu$g per ml., half of the infected cells were killed, whereas the uninfected cells were essentially unaffected. Moreover, no lysis of the cells that could be described as toxicity of melittin due to lysis at a critical concentration was observed in the concentrations used. The effect of melittin analogues showed a steady decline in the survival of infected cells by increasing the concentrations of the analogues. This effect of the melittin analogues is not easily explained. That is, infected cells are apparently viable to the same extent as uninfected cells and therefore would not be expected to die at a higher rate than uninfected cells if HIV release was inhibited. Moreover, the effect of melittin as an anti-viral substance acting through its amphiphilic structure was further substantiated by the effects of melittin (1-20) Figs. 18 and 19, respectively. It should be understood, in this regard, that melittin 1-20 lacks the cell binding part of the molecule. This analogue, however, notwithstanding inhibited HIV release as did the natural melittin and melittin (1-20)-6-(Gly)-Amide. Moreover this same melittin also inhibited the growth of infected cells as did the melittin without the positively charged C-terminal, that is melittin (1-20)-6-(Gly)-Amide. Therefore, the antiviral effect of melittin appears to be independent of the known cell lytic mechanisms of melittin but rather appeared dependent on the amphiphilic and membrane chaotropic effects of melittin. The selective killing of infected cells may therefore be a function of the inhibition of HIV release, although this is at the present time speculative and the effect may be due to a hitherto undiscovered effect of melittin.

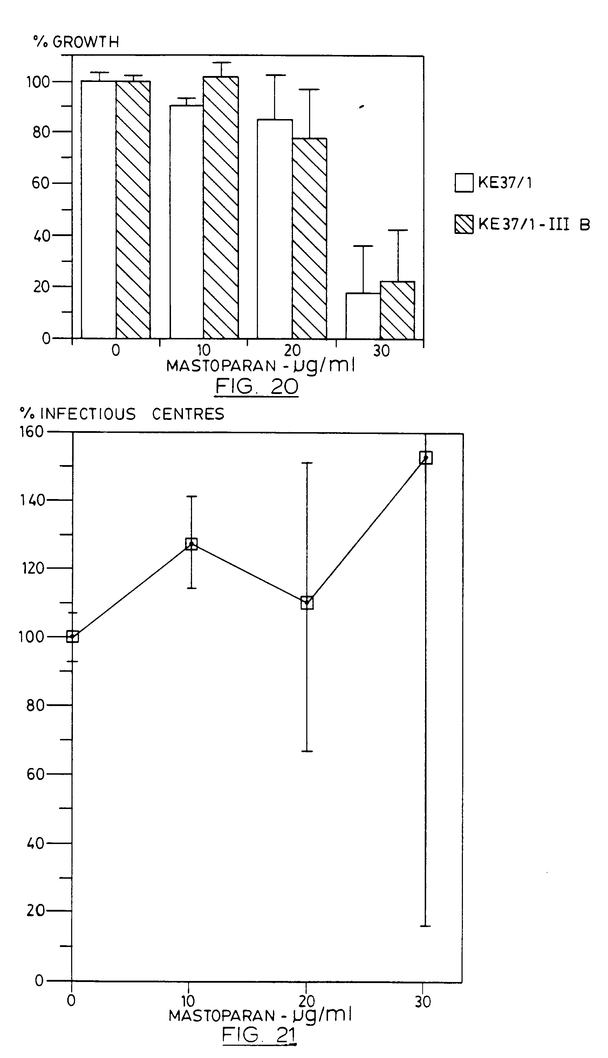

The effects of the toxin mastoparan and which are shown in Figs. 20 and 21 were also explored. This is also an amphiphilic peptide but it is considerably shorter and contains only 14 amino acid residues. Therefore, it is only capable of spanning the membrane in a polymerized form. The results are summarized in Figs. 20 and 21. It is important to note that peptides with similar structure, that is, MHC, (MAJOR histocompatibility complex sequence), and GP41 analogues, as well as mastoparan do not have any readily apparent effects. Further, a control peptide but which has no apparent structural similarity to melittin is also without effect.

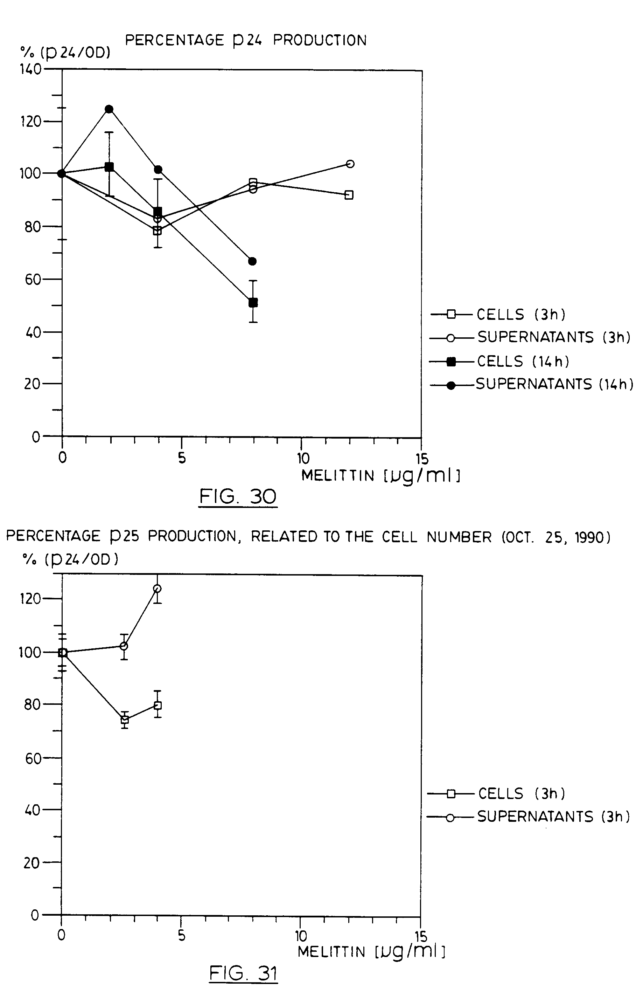

Referring more particularly now to Figs. 30 and 31, the inventors have discovered that by measuring the formation of virus protein P24 within the cell it is possible to show that the virus protein synthesis is reduced in totally infected (clone cultures) treated with melittin. This means, in essence, that the effect of melittin on the virus is to attack the virus before it is released from an infected cell although it is possible that a reduction in virus numbers may be possible during and immediately after the addition of melittin to a cell culture or otherwise. However, the present evidence indicates that virus production takes approximately 3 to 5 days to reach its maximum after initiation of the culture. At this point in time, melittin is absent from the culture. Moreover, the test results obtained show that melittin has a half-life of 19 hours and is not measurable in the medium two hours after addition to the culture, that is, it is taken up by the cells. This observation excludes any effect in the experiments to be explained by a direct effect of melittin on "cell free" virus, that is, virus released into the supernatant.

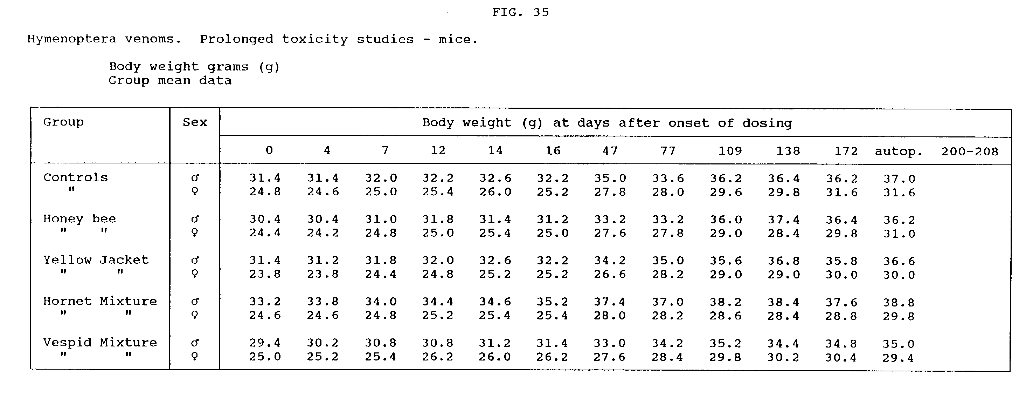

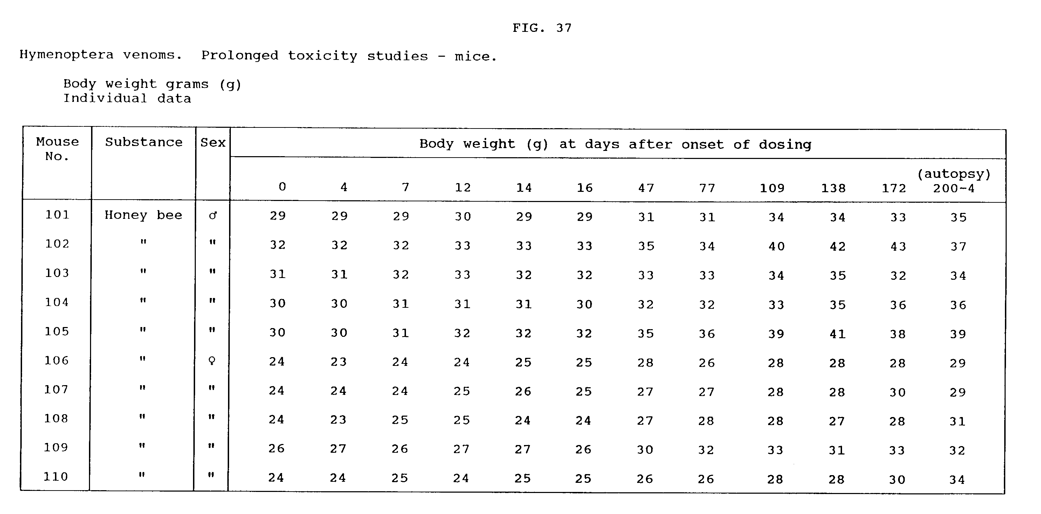

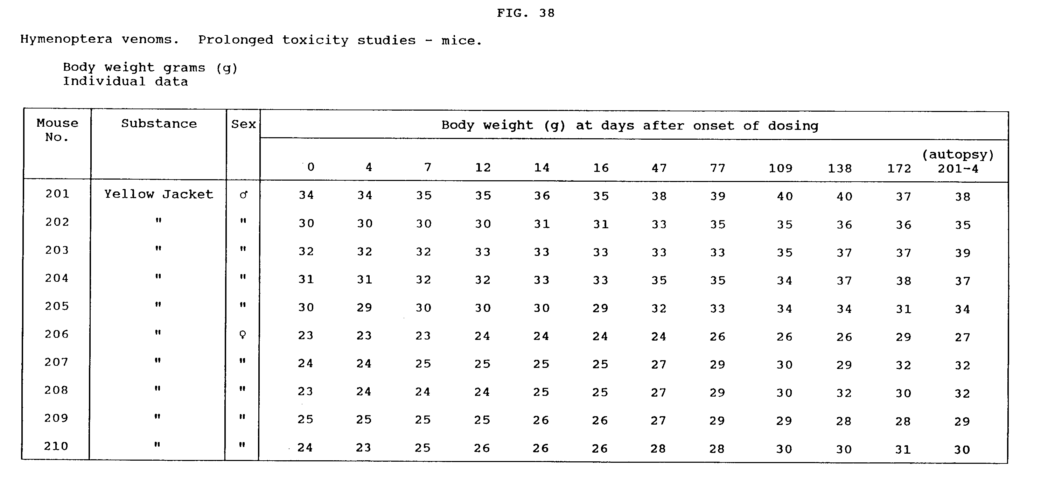

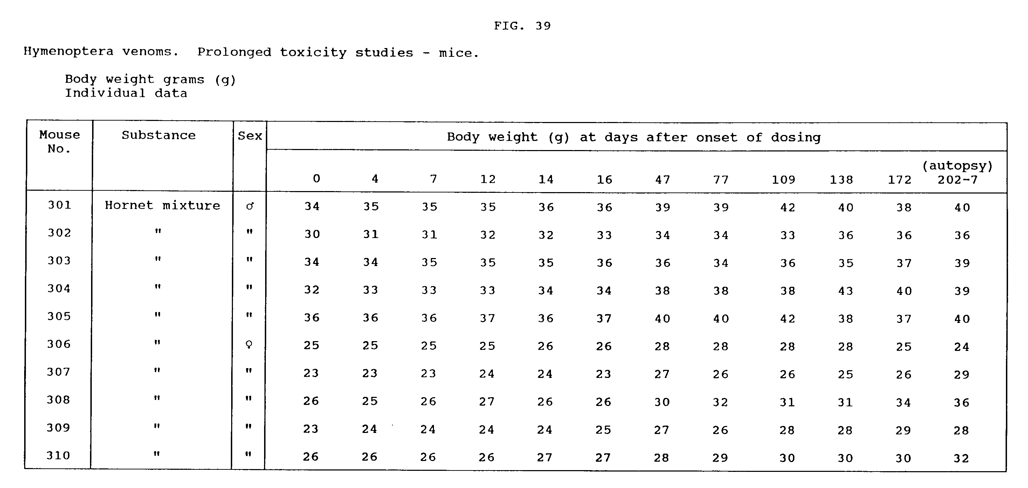

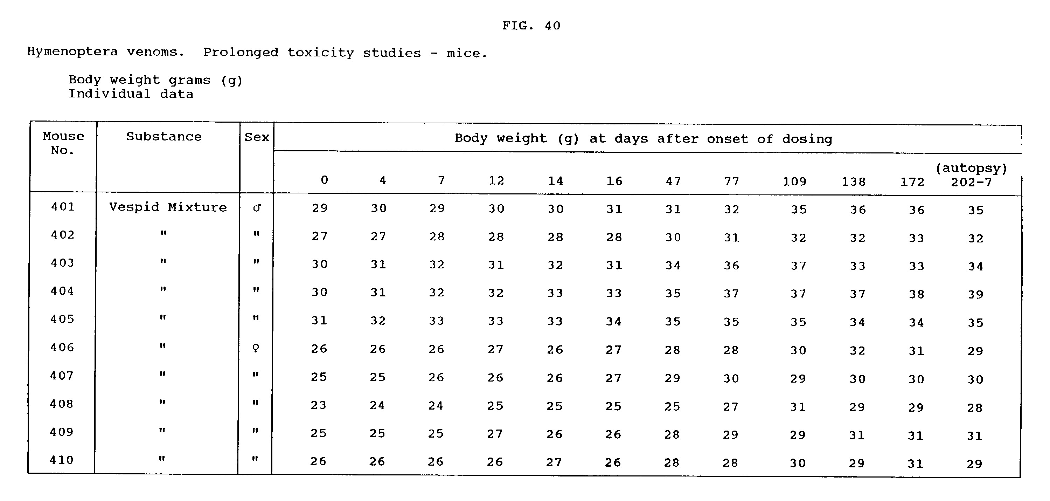

The effect of repeated administration of hymenoptera venoms was investigated in mice. Groups of 10 NMRI mice (5 of each sex) were subcutaneously given the following venoms: honey bee, yellow jacket, hornet mixture or vespid mixture days 0, 4, 7, 12, 14 and 16. Each animal was also administered 1, 2.5, 5, 10, 25 and 50$\mu$g of venom, respectively. Thereafter each animal received five monthly injections of 50$\mu$g of venom. A fifth group was given a control solution and served as controls. The data is summarized in Figs. 35 through 41A, respectively. Clinical observations and body weight registrations were repeatedly performed. At the termination of the experiment all animals were autopsied and a histopathological examination was performed. From the results of the investigation it was concluded that the mice tolerated the four venoms very well. No serious growths or microscopic changes were found.

## UTILIZING VARIOUS HYMENOPTERA VENOMS

### Introduction

To assess the effect of repeated administration of hymenoptera venoms on mammals, a prolonged toxicity study was carried out on mice.

### Test substances

Venoms from the following hymenoptera species were tested:
Honey bee (Apis mellifera) (ref. Nr. BV 02)
Yellow jacket (Vespula maculifrons) (Ref. Nr. YJ 02)
Hornet Mixture (White-faced hornet (Vespula maculata) and Yellow hornet (Vespula arenaria)) (ref. nr. MH 01).
Vespid Mixture (Yellow Jacket, White-faced hornet and Yellow hornet) (ref. No. MV 02).
The control animals received the following control solution:
NaCl: 0.12 M
Human serum albumin (HSA): 0.03%
Mannitol: 3%
Sodium phosphate: 0.005 M
pH 7.4

### Substance preparation

The venoms were diluted in Evans' buffered saline:
$Na_2HPO_4 \cdot 2H_2O$ - 0.711 g/L
$KH_2PO_4$ - 0.363 g/L
NaCl - 5 g/L
Phenol - 4 g/L

di-Na-EDTA · $2H_2O$ - 0.1 g/L
Ph 7 in different concentrations and in order to preserve the same dosage volume at each time, i.e. 0.5 ml/animal. The substances were prepared freshly at each administration.

Animals and conditions

Animals: mice, NMRI strain, SPF (Anticimex, Norrviken), seven weeks old, weighing approx. 25 g (♀) and 30 g (♂), were housed 5 animals/cage.
Diet: pellets (Anticimex, R3) and water ad libitum.
Temperature (ambient): 22 ± 1° C.
Humidity (relative): 50 ± 10%.
Light (artificial): 12 hours/day.

Group size

Ten animals (five of each sex) were allocated, by random selection, to each of the five groups (including one control group).

| Group | Mice No. | Compound |
|-------|----------|----------|
| 1 | 1 - 10 | Controls |
| 2 | 101 - 110 | Honey bee |
| 3 | 201 - 210 | Yellow Jacket |
| 4 | 301 - 310 | Hornet Mixture |
| 5 | 401 - 410 | Vespid Mixture |

Dosage

To each of the groups no. 2-5 the following dosage schedule was used:

```
Day        0        1      µg   venom/animal
 "         4        2.5     "    "      "
 "         7        5       "    "      "
 "        12       10       "    "      "
 "        14       25       "    "      "
 "        16       50       "    "      "
```

Thereafter, each mouse received five monthly injections of 50 µg of the venom. The total amount of venom injected per mouse during the experimental period was thus 343.5 µg. The control animals followed the same time schedule but received at each opportunity 0.5 ml / animal of the control solution which had been diluted in Evans' buffered saline to contain the same relative concentrations of mannitol and HSA as group 2 and 3.

Route of administration

Subcutaneously in the mid-dorsal, lumbar region.

Observations

Clinical symptoms Clinical symptoms of ill health or toxicity were controlled and recorded daily.

Body weight

Individual body weight was recorded before onset of dosing and thereafter at each administration.

Terminal studies

Six months after beginning the 50 $\mu$g injections all mice were sacrificed and autopsied. Tissues for histological preparation and histopathological examination were taken from the following organs; skin, salivary gland, trachea, lungs and bronchi, heart and aorta, thyroids, parathyroids, oesophagus, stomach, duodenum, jejunum, ileum, cecum, colon, mesenteric lymph nodes, liver, gallbladder, thigh muscle, sciatic nerve, sternebrae, thymus, pancreas, spleen, kidneys, adrenal, bladder, seminal vesicle, prostate, testes, ovaries, uterus, brain, pituitary gland, eyes, spinal chord, and injection sites.

**Results**

The results of the investigations are presented as individual data in Figs. 35-41A.

Observations

Clinical symptoms

Mouse no. 309 (Hornet Mixture) made continuous circular movements during the last two months of the experimental period.

Mouse no. 203 (Yellow Jacket) had edematous and sore fore legs with losing of the hair for a short period one month before the termination of the experiment.

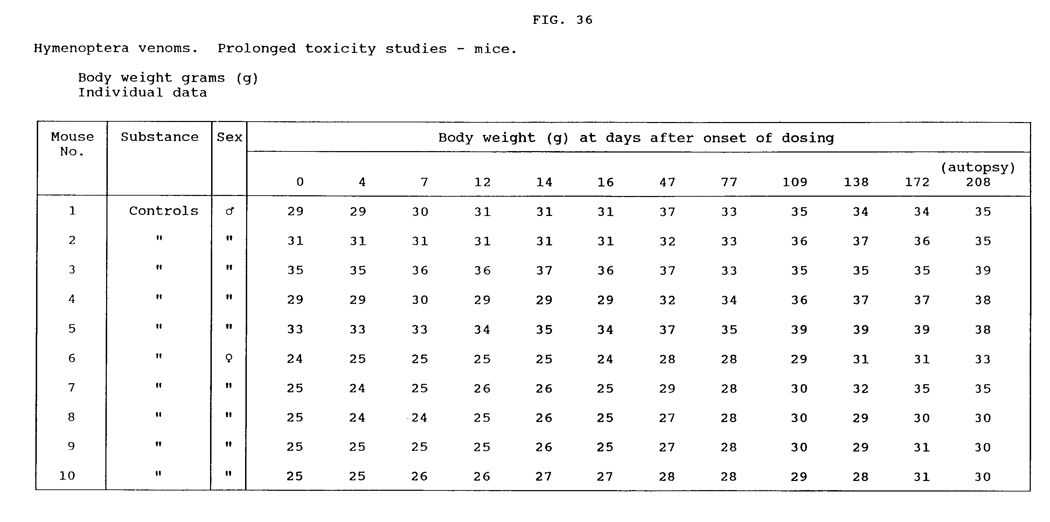

Body weight See Figs. 36-40.

Body weight was not influenced by the medication.

Terminal studies

Gross pathology

The spleen was firmly adherent to the abdominal wall in mouse no. 304 (Hornet Mixture) and to the pancreas in mouse no. 406 (Vesid Mixture). Mouse no. 101 and 105 (Honey bee) had each a firm, 1 mm nodule in their lungs. No other remarkable gross findings were made. The injection sites were at the time of sacrifice without swelling or other changes.

Microscopic pathology

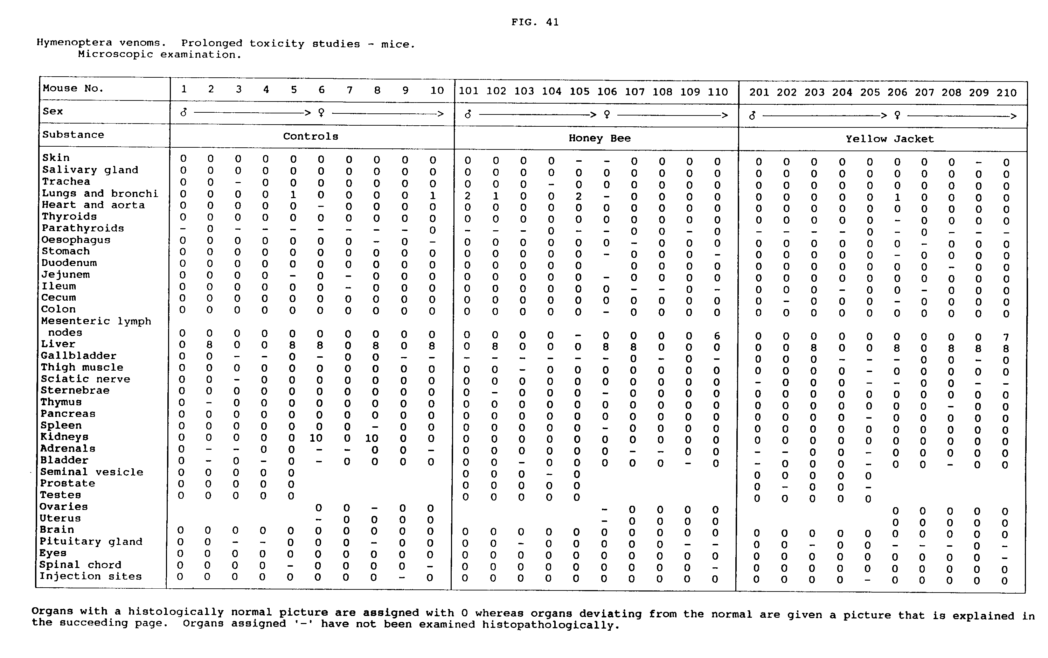

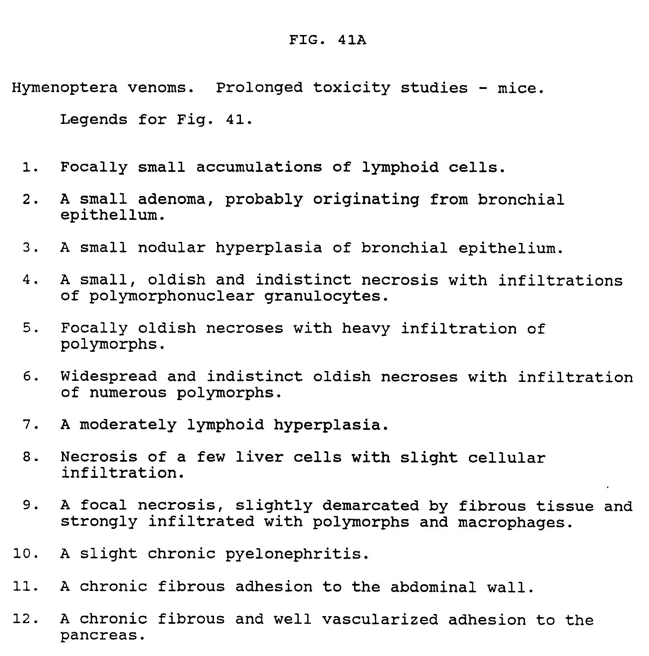

The results from the microscopic examination are presented in Figs. 41 and 41A.

Reactive necrosis in the mesenteric lymph nodes were found in the following mice: no. 110 (Honey bee), no 307 and 308 (Hornet Mixture), no 406 and 410 (Vespid Mixture). In mouse no. 210 (Yellow Jacket) there was a moderately lymphoid hyperplasia in the mesenteric lymph node.

In mouse no. 310 (Hornet Mixture) there was a focal necrosis with a subacute or chronic cellular reaction in the spleen. In mouse no. 304 (Hornet Mixture) the adhesion from the spleen to the abdominal wall was found to be of a chronic fibrous nature and the adhesion from the spleen to the pancreas in mouse no. 406 (Vespid Mixture) was chronic fibrous and well vascularized.

The described changes in the mesenteric lymph nodes and in the spleens may have been induced by the venoms but they are judged to be of an innocent character relative to the animals' condition.

Small adenomas in the lungs were seen in mouse no. 101 and 105 (Honey bee) and in mouse no. 402 (Vespid Mixture). In mouse no. 407 (Vespid Mixture) there was a small nodular hyperplasia of bronchial epithelium. These types of changes occur spontaneously.

11

In the control mice as well as the mice given the venoms, there were occasionally findings of very small necrosis of hepatic parenchymal cells with slightly cellular infiltrations. Further, slight chronic inflammatory changes were seen in the kidneys in three control mice.

In vivo experiments on Rausher leukemia mice.

The Rausher leukemia mice is a model for retroviral infections of mammals which is generally accepted. The virus produces an erythropoietic leukemia in mice which is revealed by an increase in the size of the spleen as an indication of increased production of red blood cells. The size of the spleen is taken as a measure of the progress of the disease which eventually leads to the death of the infected animals. The effect of the mellittin-analogue with a tail of 6 gly was tested in this system.

The test of the antiviral effect of the mellittin principle was carried out on Balb C mice (12 weeks old; all males). The animals were kept as 4 per cage. The animals were infected by Rausher leukemia virus ($10^5$ infectious particles (viruses) per animal in a volume of 0.2 ml) by intraperitoneal injection followed by subcutaneous injection of the test substance after 10 minutes. The animals were divided into two groups referred to as controls (receiving a sham injection of saline) or test animals (receiving the mellittin analogue).

Two groups of balb C mice each of 16 animals infected as described above were injected with either phosphate buffered saline (25 mM $KH_2PO_4$, 150 mM NaCl, pH 7.8, referred to as PBS) or with Mellittin-6-gly (5 $\mu$g/mg body weight in a volume of 0.1 ml) in PBS. The peptide was dissolved as 100 $\mu$g/ml and the controls as well as the test animals were injected with the same volume subcutaneously (0.1 ml) of PBS or PBS + test substance respectively. The injection was carried out 10 minutes after injection of the infectious virus. After 2 days the animals received a new injection of mellittin analogue (10 $\mu$g/mg body weight) or PBS as a subcutaneous injection of 0.100 ml. After 4 days from the first injection a third injection was performed using 20 $\mu$g/mg body weight of mellittin analogue. All animals were unaffected by the injections of mellittin analogue or PBS, and all survived the acute phase of the injections.

Normally infected mice will develop spleens with a weight higher than 150 mg within two weeks. The size can increase to 2.5 grams. The normal spleen is between 0.1 and 0.15 mg as lower and upper level, meaning that 95% of mice will have spleens within this range. A spleen greater than 170 mg was taken as a clear infection after two weeks, a spleen between the upper normal level of 150 mg and 170 mg as a borderline case. After 2 weeks all animals tested in the control group had developed spleens greater than 150 mg. In the test group 25% of the tested animals had also developed spleens greater than 150 mg but lower than 170 mg and were considered as borderline cases. However, the rest were showing no sign of enlargement of the spleen, that is, it was within 100-150 mg as normal animals. Only one animal had a spleen which was pathological compared to 100% in the control group.

These conclusions are drawn from these experiments.

1. The animals survive the treatment with mellittin analogues in concentrations which are comparable to the in vitro experiments described in this patent application.

2. The mellittin analogue clearly inhibits development of a retrovirus in mammals since 75% of infected animals do not develop disease compared to 100% development of disease in a comparably treated control group.

3. Not only HIV but also other retroviruses are inhibited by mellittin and its analogoues.

Conclusion

The overall impression was that the mice tolerated the four venoms, that is, Honey bee, Yellow Jacket, Hornet Mixture and Vespid Mixture very well when administered same in small dose regimens. No serious gross or microscopic changes were found.

As earlier discussed, it appears that melittin presents a method for the treatment of HIV infections in mammals by perhaps interacting with one of the major HIV proteins, that is the glycoprotein GP41. This is possible because of the similarity between the transmembrane regions of GP41 and melittin and which is best shown by reference to Fig. 5.

As shown in Fig. 5, GP41 has an amphiphilic portion which may be a prominent feature of the membrane associated part of the molecule. The amphiphilic part of the GP41 may form an antiparallel loop of charged amino acids where the two legs of the loop form a charged neutralizing structure which is similar to the structure associated with the polymerization of melittin. Melittin may also prevent the normal formation of intramolecular charge neutralization by GP41 and thereby dramatically change the structure of GP41. This may affect the formation of the virus since formation of the virus appears to be dependent on the interaction of GP41 with the nucleus of the viral protein P17, and which is able to perform the basic viral budding process. As should be understood viruses such as HIV spread from cell to cell by a process which involves formation of a small membrane bud on the cell surface and which contains the viral genome. This process seems to be sensitive to modifications of the cell surface. It is well known, for example, that addition of lipid formulations prevent the release of virus from infected cells. This effect is possibly due to a chaotropic action on the cell membrane. However, the mechanism of this action is still unclear.

The structural features of protein GP41 which permits melittin to interact with same appears to be located between amino acids 770 and 856 and involves the amphiphilic sequences 770 through 794 and 824 through 856. Please see Fig. 5. The amphiphilicity of these sequences was detected by their high hydrophobic momentum combined with a low overall hydrophobicity. The two amphiphilic legs have been modeled by molecular dynamics and they have been shown to be able to interact on the basis of their amphiphilicity and thereby potentially enable them to insert into the membrane by forming a transmembrane loop. Alternatively, they may float on the internal surface of the cell membrane. In either case, an interaction with melittin may cause a changed localization of the site of the cytoplasmeric part of GP41 thereby preventing the interaction with P17 which seems to be a necessary precursor for virus formation. In this regard, an interesting analogy between melittin and GP41 amphiphilic sequence 824 through 856 is that they both have a proline in the amphiphilic helix. This structural feature, as earlier discussed, introduces a 120° kink in the helix thus causing it to have a slightly bent form. Alternatively, melittin may interact with the membrane thus acting as a chaotropic agent. This also may be due to its amphiphilic nature. As should be understood, the effects of chaotropic agents are to cause a perturbation of the ordered phospholipid arrangement in the membrane. Such effects cause changes in the phase transition points of the membranes and subsequent changes in their thickness. As could be expected, the fact that floating melittin causes a chaotropic affect on the cell membrane is not surprising. However, and how this same event effects the budding process of the virus is unclear and not known at present. There are, however, strong indications of anti-HIV effects or changes induced in the order of the membrane lipid arrangement. Furthermore, addition of various phospholipid formulations also prevent HIV release from infected cells. It has not been possible, however, to identify the lipid species which causes for this effect.

Melittin is, however, known to interact with phospholipids, and this effect, or the binding, of an unidentified lipid species appears to be a function in HIV release and may also offer an explanation for the anti-effect of melittin. Presumably this lipid is not a phospholipid since addition of phospholipids have an effect very similar to melittin.

In conclusion, these test results appear to indicate that melittin has two effects, that is, it appears to have a first short term inactivation of the virus and a second long term reduction of at least P24 production. These characteristics appear to be due to the specific structure of the melittin amphiphilic helix. Further, the test information indicates that the tail structure determines the effectivity of an analog but not the quality of its effect. That is, the effective concentration of an analogue with a tail which includes Lys-Arg-Lys-Arg-Gly-Gly at its tail is about a hundred times lower than the effective concentration of no-tail melittin. In addition to the foregoing, the test results indicate that supernatant infectivity was reduced following a single treatment with melittin at subtoxic concentrations. In this regard, treatment was a cultivation start and measurement of infectivity was seven days post treatment. As was discussed earlier, maximum virus concentration in cultures of this type is not achieved for five days after cultivation. As should be understood and the conclusions which the inventor have drawn from same, is that if the reduced viral infectivity seven days post treatment were only due to direct effects of melittin on the virus, then in that event the melittin or an active fragment would have to be present as late as five days post treatment. This is not possible inasmuch as the inventors have discovered that melittin is absorbed by the cell within hours after introduction to the culture. Moreover, and if the reduction and supernatant infectivity was partly due to reduced virus production as opposed to incorrect virus assembly or destruction of newly synthesized virion, then, in that event, this should be reflected by reduced amounts of viral proteins within the cells and/or in their supernatants of the melittin treated cultures. To determine whether this hypothesis is correct the inventor chose the P24 marker as a marker for viral protein production. The test data shows that there is if at all only a slight reduction of cellular and supernatant P24 following three hours of incubation with melittin. However, following fourteen hours of incubation with melittin, there is a marked reduction of P24 within the cells as well as in the supernatant (down to 60%). Therefore, cellular P24 seems to be reduced sooner and more strongly than supernatant P24. Moreover, incubation of cell free HIV with melittin does not impair the detection of the P24 antigen. It rather only appears to cause the virus infectivity to be reduced.

In vitro experiments on mice were also performed. The following conclusions were drawn:

1. The animals survive the treatment with mellittin analogues in concentrations which are comparable to the in vitro experiments described in this patent application.

EP 0 504 191 B1

2. The mellittin analogue clearly inhibits development of a retrovirus in mammals since 75% of infected animals do not develop disease compared to 100% development of disease in a comparably treated control group.

3. Not only HIV but also other retroviruses are inhibited by mellittin and its analogoues.

**Claims**

1. Use of an effective subtoxic dosage of melittin in the manufacture of a medicament for the treatment of an HIV infection in a mammal whereby virus replication in the HIV infected cells of a mammal is reduced or inhibited.

2. Use of an effective subtoxic dosage of a structural analogue of melittin in the manufacture of a medicament as claimed in claim 1.

3. Use of an effective subtoxic dosage of a polypeptide mixture of melittin and structural analogue thereof in the manufacture of a medicament as claimed in claim 1.

4. Use of an effective subtoxic dosage of an agent consisting of at least one hymenoptera venom, at least one active protein component of a hymenoptera venom, at least one polypeptide component of a hymenoptera venom, and/or mixtures thereof in the manufacture of a medicament as claimed in claim 1.

5. Use of an effective subtoxic dosage of honeybee venom, bumble bee venom, yellow jacket venom, bald-faced hornet venom, active protein components of said venom, active polypeptide components of said venom, and mixtures thereof in the manufacture of a medicament as claimed in claim 1.

6. Use of an effective subtoxic dosage of an amphiphilic $\alpha$ helix with or without an attached cell binding sequence in the manufacture of a medicament as claimed in claim 1.

7. Use of an effective subtoxic dosage of

```
                                    Gly-Ile-
       Gly-Ala-Val-Leu-Lys-Val-Leu-Thr-Thr-Gly-Leu-Pro-Ala-
       Leu-Ile-Ser-Trp-Ile-Gly-Gly-Gly-Gly-Gly-Gly
```

in the manufacture of a medicament as claimed in claim 1.

8. Use of an effective subtoxic dosage of melittin and the structural analogues of

```
                              Gly-Ile-Gly-Ala-Val-
       Leu-Lys-Val-Leu-Thr-Thr-Gly-Leu-Pro-Ala-Leu-Ile-Ser-
       Trp-Ile-Gly-Gly-Gly-Gly-Gly-Gly
```

in the manufacture of a medicament as claimed in claim 1.

9. Use of an effective subtoxic dosage of melittin in the manufacture of a medicament for the treatment of an HIV infection in a mammal whereby growth of HIV infected cells in the mammal is reduced or inhibited.

10. Use of an effective subtoxic dosage of a structural analogue of melittin in the manufacture of a medicament as claimed in claim 9.

11. Use of Amfi 1 and peptides of GP41 that are melittin-like in the manufacture of a medicament as claimed in claim 9.

14

**12.** Use of Amfi 2 and peptides of GP41 that are melittin-like in the manufacture of a medicament as claimed in claim 9.

**13.** Use of an effective, subtoxic dosage of a polypeptide mixture of melittin and structural analogue thereof in the manufacture of a medicament as claimed in claim 9.

**14.** Use of an effective subtoxic dosage of an agent consisting of at least one hymenoptera venom, at least one active protein component of a hymenoptera venom, at least one polypeptide component of a hymenoptera venom, and/or mixtures thereof in the manufacture of a medicament as claimed in claim 9.

**15.** Use of an effective dosage of an agent consisting essentially of:
honeybee venom, bumble bee venom, yellow jacket venom, bald faced hornet venom, active protein components of said venom, active polypeptide components of said venom, and/or mixtures thereof in the manufacture of a medicament as claimed in claim 9.

**16.** Use of an effective dosage of an amphiphilic $\alpha$ helix with or without an attached cell binding sequence in the manufacture of a medicament as claimed in claim 9.

**17.** Use of an effective dosage of

<div align="center">

Gly-Ile-Gly-Ala-Val-Leu-Lys-Val-Leu-Thr-Thr-Gly-Leu-Pro-Ala-Leu-Ile-Ser-Trp-Ile-Gly-Gly-Gly-Gly-Gly-Gly

</div>

in the manufacture of a medicament as claimed in claim 9.

**18.** Use of an effective dosage of melittin and the structural analogue

<div align="center">

Gly-Ile-Gly-Ala-Val-Leu-Lys-Val-Leu-Thr-Thr-Gly-Leu-Pro-Ala-Leu-Ile-Ser-Trp-Ile-Gly-Gly-Gly-Gly-Gly-Gly

</div>

in the manufacture of a medicament as claimed in claim 9.

**19.** Use of an effective subtoxic dosage of melittin in the manufacture of a medicament for the treatment of a retroviral infection in a mammal whereby virus replication in the retroviral infected cells of the mammal is reduced or inhibited.

**20.** Use of an effective subtoxic dosage of a structural analogue of melittin in the manufacture of a medicament as claimed in claim 19.

**21.** Use of an effective, subtoxic dosage of a polypeptide mixture of melittin and structural analogue thereof in the manufacture of a medicament as claimed in claim 19.

**22.** Use of an effective subtoxic dosage of an agent consisting of at least one hymenoptera venom, at least one active protein component of a hymenoptera venom, at least one polypetide component of a hymenoptera venom, and/or mixtures thereof in the manufacture of a medicament as claimed in claim 19.

**23.** Use of an effective subtoxic dosage of an agent consisting essentially of:
honeybee venom, bumble bee venom, yellow jacket venom, bald-faced hornet venom active protein components of said venom, active polypeptide components of said venom, and/or mixtures thereof in the manufacture of a medicament as claimed in claim 19.

**24.** Use of an effective subtoxic dosage of an amphiphilic α helix with or without an attached cell binding sequence in the manufacture of a medicament as claimed in claim 19.

**25.** Use of an effective dosage of

$$Gly-Ile-Gly-Ala-Val-Leu-Lys-Val-Leu-Thr-Thr-Gly-Leu-Pro-Ala-Leu-Ile-Ser-Trp-Ile-Gly-Gly-Gly-Gly-Gly-Gly$$

in the manufacture of a medicament as claimed in claim 19.

**26.** Use of an effective dosage of melittin and the structural analogue

$$Gly-Ile-Gly-Ala-Val-Leu-Lys-Val-Leu-Thr-Thr-Gly-Leu-Pro-Ala-Leu-Ile-Ser-Trp-Ile-Gly-Gly-Gly-Gly-Gly-Gly$$

in the manufacture of a medicament as claimed in claim 19.

**27.** Use of an effective subtoxic dosage of melittin in the manufacture of a medicament for the treatment of a retroviral infection in a mammal whereby growth of retroviral infected cells in the mammal is reduced or inhibited.

**28.** Use of an effective subtoxic dosage of a structural analogue of melittin in the manufacture of a medicament as claimed in claim 27.

**29.** Use of Amfi 1 and peptides of GP41 that are melittin-like in the manufacture of a medicament as claimed in claim 27.

**30.** Use of Amfi 2 and peptides of GP41 that are melittin-like in the manufacture of a medicament as claimed in claim 27.

**31.** Use of an effective, subtoxic dosage of a polypeptide mixutre of melittin and structural analogue thereof in the manufacture of a medicament as claimed in claim 27.

**32.** Use of an effective subtoxic dosage of an agent consisting of at least one hymenoptery venom, at least one active protein component of a hymenoptera venom, at least one polypeptide component of a hymenoptera venom, and/or mixtures thereof in the manufacture of a medicament as claimed in claim 27.

**33.** Use of an agent consisting essentially of:
honeybee venom, bumble bee venom, yellow jacket venom, bald-faced hornet venom active protein components of said venom, active polypeptide components of said venom, and/or mixtures thereof in the manufacture of a medicament as claimed in claim 27.

**34.** Use of an amphiphilic α helix with or without an attached cell binding sequence in the manufacture of a medicament as claimed in claim 27.

EP 0 504 191 B1

**35.** Use of

```
Gly-Ile-Gly-Ala-Val-Leu-Lys-Val-Leu-
Thr-Thr-Gly-Leu-Pro-Ala-Leu-Ile-Ser-Trp-Ile-Gly-Gly-
Gly-Gly-Gly-Gly
```

in the manufacture of a medicament as claimed in claim 27.

**36.** Use of melittin and the structural analogue

```
Gly-Ile-Gly-Ala-Val-Leu-Lys-Val-Leu-Thr-Thr-Gly-Leu-
Pro-Ala-Leu-Ile-Ser-Trp-Ile-Gly-Gly-Gly-Gly-Gly-Gly
```

in the manufacture of a medicament as claimed in claim 27.

**Patentansprüche**

1. Verwendung einer wirksamen subtoxischen Dosierung von Melittin bei der Herstellung eines Medikaments zur Behandlung einer HIV-Infektion bei einem Säuger, wodurch die Virusreplikation in den mit HIV infizierten Zellen des Säugers verringert oder gehemmt wird.

2. Verwendung einer wirksamen subtoxischen Dosierung eines strukturellen Analogon von Melittin bei der Herstellung eines Medikaments nach Anspruch 1.

3. Verwendung einer wirksamen subtoxischen Dosierung einer Polypeptidmischung von Melittin und eines strukturellen Analogon davon bei der Herstellung eines Medikaments nach Anspruch 1.

4. Verwendung einer wirksamen subtoxischen Dosierung eines Mittels, das aus mindestens einem Tiergift von Hymenoptera, mindestens einer aktiven Proteinkomponente eines Tiergifts von Hymenoptera, mindestens einer Polypeptidkomponente eines Tiergifts von Hymenoptera und/oder Mischungen davon besteht, bei der Herstellung eines Medikaments nach Anspruch 1.

5. Verwendung einer wirksamen subtoxischen Dosierung von Bienengift, Hummelgift, Wespengift, Gift der kahlen Hornisse, wirksamen Proteinkomponenten dieses Tiergifts, wirksamen Polypeptidkomponenten dieses Tiergifts und Mischungen davon bei der Herstellung eines Medikaments nach Anspruch 1.

6. Verwendung einer wirksamen subtoxischen Dosierung einer amphiphilen $\alpha$-Helix mit oder ohne angefügte Zellbindungssequenz bei der Herstellung eines Medikaments nach Anspruch 1.

7. Verwendung einer wirksamen subtoxischen Dosierung von

```
Gly-Ile-Gly-Ala-Val-Leu-Lys-Val-Leu-Thr-Thr-Gly-Leu-Pro-
Ala-Leu-Ile-Ser-Trp-Ile-Gly-Gly-Gly-Gly-Gly-Gly
```

bei der Herstellung eines Medikaments nach Anspruch 1.

17

8. Verwendung einer wirksamen subtoxischen Dosierung von Melittin und strukturellen Analoga mit

```
            Gly-Ile-Gly-Ala-Val-
    Leu-Lys-Val-Leu-Thr-Thr-Gly-Leu-Pro-Ala-Leu-Ile-Ser-Trp-
    Ile-Gly-Gly-Gly-Gly-Gly-Gly
```

bei der Herstellung eines Medikaments nach Anspruch 1.

9. Verwendung einer wirksamen subtoxischen Dosierung von Melittin bei der Herstellung eines Medikaments zur Behandlung einer HIV-Infektion beim Säuger, wodurch das Wachstum der mit HIV infizierten Zellen im Säuger verringert oder gehemmt wird.

10. Verwendung einer wirksamen subtoxischen Dosierung eines strukturellen Analogon von Melittin bei der Herstellung eines Medikaments nach Anspruch 9.

11. Verwendung von Amfi 1 und Peptiden von GP41, die dem Melittin ähnlich sind, bei der Herstellung eines Medikaments nach Anspruch 9.

12. Verwendung von Amfi 2 und Peptiden von GP41, die dem Melittin ähnlich sind, bei der Herstellung eines Medikaments nach Anspruch 9.

13. Verwendung einer wirksamen subtoxischen Dosierung einer Polypeptidmischung von Melittin und eines strukturellen Analogon davon bei der Herstellung eines Medikaments nach Anspruch 9.

14. Verwendung einer wirksamen subtoxischen Dosierung eines Mittels, das aus mindestens einem Tiergift der Hymenoptera, mindestens einer aktiven Proteinkomponente eines Tiergifts der Hymenoptera, mindestens einer Polypeptidkomponente eines Tiergifts der Hymenoptera und/oder Mischungen davon besteht, bei der Herstellung eines Medikaments nach Anspruch 9.

15. Verwendung einer wirksamen Dosierung eines Mittels, das im wesentlichen aus Bienengift, Hummelgift, Wespengift, Gift der kahlen Hornisse, wirksamen Proteinkomponenten dieses Tiergifts, wirksamen Polypeptidkomponenten dieses Tiergifts und/oder Mischungen davon besteht, bei der Herstellung eines Medikaments nach Anspruch 9.

16. Verwendung einer wirksamen Dosierung einer amphiphilen $\alpha$-Helix mit oder ohne angefügte Zellbindungssequenz bei der Herstellung eines Medikaments nach Anspruch 9.

17. Verwendung einer wirksamen Dosierung von

```
            Gly-Ile-Gly-Ala-
    Val-Leu-Lys-Val-Leu-Thr-Thr-Gly-Leu-Pro-Ala-Leu-Ile-Ser-
    Trp-Ile-Gly-Gly-Gly-Gly-Gly-Gly
```

bei der Herstellung eines Medikaments nach Anspruch 9.

18. Verwendung einer wirksamen Dosierung von Melittin und des strukturellen Analogon

```
            Gly-Ile-Gly-Ala-Val-Leu-Lys-Val-
    Leu-Thr-Thr-Gly-Leu-Pro-Ala-Leu-Ile-Ser-Trp-Ile-Gly-Gly-
    Gly-Gly-Gly-Gly
```

bei der Herstellung eines Medikaments nach Anspruch 9.

18

**19.** Verwendung einer wirksamen subtoxischen Dosierung von Melittin bei der Herstellung eines Medikaments zur Behandlung einer Retrovirus-Infektion beim Säuger, wodurch die Replikation des Virus in den mit Retroviren infizierten Zellen des Säugers verringert oder gehemmt wird.

**20.** Verwendung einer wirksamen subtoxischen Dosierung eines strukturellen Analogon von Melittin bei der Herstellung eines Medikaments nach Anspruch 19.

**21.** Verwendung einer wirksamen subtoxischen Dosierung einer Polypeptidmischung von Melittin und eines strukturellen Analogon davon bei der Herstellung eines Medikaments nach Anspruch 19.

**22.** Verwendung einer wirksamen subtoxischen Dosierung eines Mittels, das aus mindestens einem Tiergift der Hymenoptera, mindestens einer aktiven Proteinkomponente eines Tiergifts der Hymenoptera, mindestens einer Polypeptidkomponente eines Tiergifts der Hymenoptera und/oder Mischungen davon besteht, bei der Herstellung eines Medikaments nach Anspruch 19.

**23.** Verwendung einer wirksamen subtoxischen Dosierung eines Mittels, das im wesentlichen aus Bienengift, Hummelgift, Wespengift, Gift der kahlen Hornisse, wirksamen Proteinkomponenten dieses Gifts, wirksamen Polypeptidkomponenten dieses Gifts und/oder Mischungen davon besteht, bei der Herstellung eines Medikaments nach Anspruch 19.

**24.** Verwendung einer wirksamen subtoxischen Dosierung einer amphiphilen $\alpha$-Helix mit oder ohne angefügte Zellbindungssequenz bei der Herstellung eines Medikaments nach Anspruch 19.

**25.** Verwendung einer wirksamen Dosierung von

```
                              Gly-Ile-Gly-Ala-
        Val-Leu-Lys-Val-Leu-Thr-Thr-Gly-Leu-Pro-Ala-Leu-Ile-Ser-
        Trp-Ile-Gly-Gly-Gly-Gly-Gly-Gly
```

bei der Herstellung eines Medikaments nach Anspruch 19.

**26.** Verwendung einer wirksamen Dosierung von Melittin und des strukturellen Analogon

```
                          Gly-Ile-Gly-Ala-Val-Leu-Lys-Val-
        Leu-Thr-Thr-Gly-Leu-Pro-Ala-Leu-Ile-Ser-Trp-Ile-Gly-Gly-
        Gly-Gly-Gly-Gly
```

bei der Herstellung eines Medikaments nach Anspruch 19.

**27.** Verwendung einer wirksamen subtoxischen Dosierung von Melittin bei der Herstellung eines Medikaments zur Behandlung einer Retrovirus-Infektion beim Säuger, wodurch das Wachstum der mit Retroviren infizierten Zellen beim Säuger verringert oder gehemmt wird.

**28.** Verwendung einer wirksamen subtoxischen Dosierung eines strukturellen Analogon von Melittin bei der Herstellung eines Medikaments nach Anspruch 27.

**29.** Verwendung von Amfi 1 und Peptiden von GP41, die dem Melittin ähnlich sind, bei der Herstellung eines Medikaments nach Anspruch 27.

**30.** Verwendung von Amfi 2 und Peptiden von GP41, die dem Melittin ähnlich sind, bei der Herstellung eines Medikaments nach Anspruch 27.

EP 0 504 191 B1

**31.** Verwendung einer wirksamen subtoxischen Dosierung einer Polypeptidmischung von Melittin und einem strukturellen Analogon davon bei der Herstellung eines Medikaments nach Anspruch 27.

**32.** Verwendung einer wirksamen subtoxischen Dosierung eines Mittels, das aus mindestens einem Tiergift der Hymenoptera, mindestens einer aktiven Proteinkomponente eines Tiergifts der Hymenoptera, mindestens einer Polypeptidkomponente eines Tiergifts der Hymenoptera und/oder Mischungen davon besteht, bei der Herstellung eines Medikaments nach Anspruch 27.

**33.** Verwendung eines Mittels, das im wesentlichen aus Bienengift, Hummelgift, Wespengift, Gift der kahlen Hornisse, wirksamen Proteinkomponenten dieses Gifts, wirksamen Polypeptidkomponenten dieses Gifts und/oder Mischungen davon besteht, bei der Herstellung eines Medikaments nach Anspruch 27.

**34.** Verwendung einer amphiphilen α-Helix mit oder ohne angefügte Zellbindungssequenz bei der Herstellung eines Medikaments nach Anspruch 27.

**35.** Verwendung von

```
Gly-Ile-Gly-Ala-Val-Leu-Lys-Val-Leu-Thr-
Thr-Gly-Leu-Pro-Ala-Leu-Ile-Ser-Trp-Ile-Gly-Gly-Gly-Gly-
Gly-Gly
```

bei der Herstellung eines Medikaments nach Anspruch 27.

**36.** Verwendung von Melittin und einem strukturellen Analogon

```
Gly-Ile-Gly-Ala-Val-Leu-Lys-Val-Leu-Thr-Thr-Gly-Leu-Pro-
Ala-Leu-Ile-Ser-Trp-Ile-Gly-Gly-Gly-Gly-Gly-Gly
```

bei der Herstellung eines Medikaments nach Anspruch 27.

**Revendications**

**1.** Utilisation d'une dose sub-toxique efficace de melittine dans la fabrication d'un médicament pour le traitement d'une infection par le HIV chez un mammifère, dans lequel la réplication virale dans les cellules du mammifère infectées par le HIV est réduite ou inhibée.

**2.** Utilisation d'une dose sub-toxique efficace d'un analogue structurel de la melittine dans la fabrication d'un médicament ainsi que revendiqué dans la revendication 1.

**3.** Utilisation d'une dose sub-toxique efficace d'un mélange polypeptidique de melittine et d'un de ses analogues structurels dans la fabrication d'un médicament ainsi que revendiqué dans la revendication 1.

**4.** Utilisation d'une dose sub-toxique efficace d'un agent consistant en au moins un venin d'hyménoptères, au moins un composant protéique actif d'un venin d'hyménoptères, au moins un composant polypeptidique d'un venin hyménoptère, et/ou leurs mélanges, dans la fabrication d'un médicament ainsi que revendiqué dans la revendication 1.

**5.** Utilisation d'une dose sub-toxique efficace de
   - venin d'abeilles,
   - venin de bourdons,
   - venin de petites guêpes,
   - venin de frelon,

20

- composants protéiques actifs desdits venins,
- composants protéiques actifs dudit venin
  et/ou leurs mélanges,

dans la fabrication d'un médicament ainsi que revendiqué dans la revendication 1.

6. Utilisation d'une dose sub-toxique efficace d'une hélice α amphiphilique avec ou sans une séquence de liaison de cellules liée dans la fabrication d'un médicament ainsi que revendiqué dans la revendication 1.

7. Utilisation d'une dose sub-toxique efficace de

```
Gly-Ile-Gly-Ala-Val-Leu-Lys-Val-Leu-Thr-Thr-Gly-Leu-Pro-Ala-
Leu-Ile-Ser-Trp-Ile-Gly-Gly-Gly-Gly-Gly-Gly
```

dans la fabrication d'un médicament ainsi que revendiqué dans la revendication 1.

8. Utilisation d'une dose sub-toxique efficace de melittine et d'analogues structurels de

```
                                            Gly-Ile-Gly-Ala-Val-
Leu-Lys-Val-Leu-Thr-Thr-Gly-Leu-Pro-Ala-Leu-Ile-Ser-Trp-Ile-
Gly-Gly-Gly-Gly-Gly-Gly
```

dans la fabrication d'un médicament ainsi que revendiqué dans la revendication 1.

9. Utilisation d'une dose sub-toxique efficace de melittine dans la fabrication d'un médicament pour le traitement d'une infection par le HIV chez un mammifère, la croissance des cellules infectées par le HIV chez le mammifère étant réduite ou inhibée.

10. Utilisation d'une dose sub-toxique efficace d'un analogue structurel de la melittine dans la fabrication d'un médicament ainsi que revendiqué dans la revendication 9.

11. Utilisation d'Amfi 1 et des peptides de GP41 qui sont similaires à la melittine dans la fabrication d'un médicament ainsi que revendiqué dans la revendication 9.

12. Utilisation d'Amfi 2 et des peptides de GP41 qui sont similaires à la melittine dans la fabrication d'un médicament ainsi que revendiqué dans la revendication 9.

13. Utilisation d'une dose sub-toxique efficace d'un mélange polypeptidique de melittine et de ses analogues structurels, dans la fabrication d'un médicament ainsi que revendiqué dans la revendication 9.

14. Utilisation d'une dose sub-toxique efficace d'un agent consistant en au moins un venin d'hyménoptères, au moins un composant protéique actif d'un venin d'hyménoptères, au moins un composant polypeptidique d'un venin d'hyménoptères, et/ou leurs mélanges, dans la fabrication d'un médicament ainsi que revendiqué dans la revendication 9.

15. Utilisation d'une dose sub-toxique efficace de
   - venin d'abeilles,
   - venin de bourdons,
   - venin de petites guêpes,
   - venin de frelon,
   - composants protéiques actifs desdits venins,
   - composants protéiques actifs dudit venin
     et/ou leurs mélanges,

dans la fabrication d'un médicament ainsi que revendiqué dans la revendication 9.

16. Utilisation d'une dose sub-toxique efficace d'une hélice α amphiphilique avec ou sans une séquence de liaison de cellules liées dans la fabrication d'un médicament ainsi que revendiqué dans la revendication 9.

17. Utilisation d'une dose sub-toxique efficace de

```
Gly-Ile-Gly-Ala-Val-Leu-Lys-Val-Leu-Thr-Thr-Gly-Leu-Pro-Ala-
Leu-Ile-Ser-Trp-Ile-Gly-Gly-Gly-Gly-Gly-Gly
```

dans la fabrication d'un médicament ainsi que revendiqué dans la revendication 9.

18. Utilisation d'une dose sub-toxique efficace de melittine et des analogues structurels

```
                                        Gly-Ile-Gly-Ala-Val-Leu-
Lys-Val-Leu-Thr-Thr-Gly-Leu-Pro-Ala-Leu-Ile-Ser-Trp-Ile-Gly-
Gly-Gly-Gly-Gly-Gly
```

dans la fabrication d'un médicament ainsi que revendiqué dans la revendication 9.

19. Utilisation d'une dose sub-toxique efficace de melittine dans la fabrication d'un médicament pour le traitement d'une infection due à un retrovirus chez un mammifère, la croissance des cellules infectées par le HIV chez le mammifère étant réduite ou inhibée.

20. Utilisation d'une dose sub-toxique efficace d'un analogue structurel de la melittine dans la fabrication d'un médicament ainsi que revendiqué dans la revendication 19.

21. Utilisation d'une dose sub-toxique efficace d'un mélange polypeptidique de melittine et de ses analogues structurels, dans la fabrication d'un médicament ainsi que revendiqué dans la revendication 19.

22. Utilisation d'une dose sub-toxique efficace d'un agent consistant en au moins un venin d'hyménoptères, au moins un composant protéique actif d'un venin d'hyménoptères, au moins un composant polypeptidique d'un venin d'hyménoptères, et/ou leurs mélanges, dans la fabrication d'un médicament ainsi que revendiqué dans la revendication 19.

23. Utilisation d'une dose sub-toxique efficace de
    - venin d'abeilles,
    - venin de bourdons,
    - venin de petites guêpes,
    - venin de frelon,
    - composants protéiques actifs desdits venins,
    - composants protéiques actifs dudit venin
      et/ou leurs mélanges,
    dans la fabrication d'un médicament ainsi que revendiqué dans la revendication 19.

24. Utilisation d'une dose sub-toxique efficace d'une hélice α amphiphilique avec ou sans une séquence de liaison de cellules liées dans la fabrication d'un médicament ainsi que revendiqué dans la revendication 19.

**25.** Utilisation d'une dose sub-toxique efficace de

```
                                                    Gly-
Ile-Gly-Ala-Val-Leu-Lys-Val-Leu-Thr-Thr-Gly-Leu-Pro-Ala-Leu-
Ile-Ser-Trp-Ile-Gly-Gly-Gly-Gly-Gly-Gly
```

dans la fabrication d'un médicament ainsi que revendiqué dans la revendication 19.

**26.** Utilisation d'une dose sub-toxique efficace de melittine et des analogues structurels

```
                                    Gly-Ile-Gly-Ala-Val-
Leu-Lys-Val-Leu-Thr-Thr-Gly-Leu-Pro-Ala-Leu-Ile-Ser-Trp-
Ile-Gly-Gly-Gly-Gly-Gly-Gly
```

dans la fabrication d'un médicament ainsi que revendiqué dans la revendication 19.

**27.** Utilisation d'une dose sub-toxique efficace de melittine dans la fabrication d'un médicament pour le traitement d'une infection due à un retrovirus chez un mammifère, la croissance des cellules infectées par le HIV chez le mammifère étant réduite ou inhibée.

**28.** Utilisation d'une dose sub-toxique efficace d'un analogue structurel de la melittine dans la fabrication d'un médicament ainsi que revendiqué dans la revendication 27.

**29.** Utilisation d'Amfi 1 et des peptides de GP41 qui sont similaires à la melittine dans la fabrication d'un médicament ainsi que revendiqué dans la revendication 27.

**30.** Utilisation d'Amfi 2 et des peptides de GP41 qui sont similaires à la melittine dans la fabrication d'un médicament ainsi que revendiqué dans la revendication 27.

**31.** Utilisation d'une dose sub-toxique efficace d'un mélange polypeptidique de melittine et de ses analogues structurels, dans la fabrication d'un médicament ainsi que revendiqué dans la revendication 27.

**32.** Utilisation d'une dose sub-toxique efficace d'un agent consistant en au moins un venin d'hyménoptères, au moins un composant protéique actif d'un venin d'hyménoptères, au moins un composant polypeptidique d'un venin d'hyménoptères, et/ou leurs mélanges, dans la fabrication d'un médicament ainsi que revendiqué dans la revendication 27.

**33.** Utilisation d'une dose sub-toxique efficace de
   - venin d'abeilles,
   - venin de bourdons,
   - venin de petites guêpes,
   - venin de frelon,
   - composants protéiques actifs desdits venins,
   - composants protéiques actifs dudit venin
      et/ou leurs mélanges,
dans la fabrication d'un médicament ainsi que revendiqué dans la revendication 27.

**34.** Utilisation d'une dose sub-toxique efficace d'une hélice $\alpha$ amphiphilique avec ou sans une séquence de liaison de cellules liées dans la fabrication d'un médicament ainsi que revendiqué dans la revendication 27.

**35.** Utilisation d'une dose sub-toxique efficace de

```
                                                            Gly-
Ile-Gly-Ala-Val-Leu-Lys-Val-Leu-Thr-Thr-Gly-Leu-Pro-Ala-Leu-
Ile-Ser-Trp-Ile-Gly-Gly-Gly-Gly-Gly-Gly
```

dans la fabrication d'un médicament ainsi que revendiqué dans la revendication 27.

**36.** Utilisation d'une dose sub-toxique efficace de melittine et des analogues structurels

```
                                      Gly-Ile-Gly-Ala-Val-
Leu-Lys-Val-Leu-Thr-Thr-Gly-Leu-Pro-Ala-Leu-Ile-Ser-Trp-Ile-
Gly-Gly-Gly-Gly-Gly-Gly
```

dans la fabrication d'un médicament ainsi que revendiqué dans la revendication 27.

24

FIG. 1

FIG. 2

FIG. 3

FIG. 4

MODEL OF THE CARBOXYTERMINAL REGION OF GP41

FIG. 5

FIG. 6

FIG. 7

% GROWTH

FIG. 8

% INFECTIOUS CENTRES

FIG. 9

29

FIG. 10

FIG. 11

FIG. 12

FIG. 13

FIG. 14

FIG. 15

% GROWTH

☐ KE37/1

▨ KE37/1-III B

MELITTIN 3 - µg/ml

FIG. 16

% INFECTIOUS CENTRES

MELITTIN 3 - µg/ml

FIG. 17

FIG. 18

FIG. 19

FIG. 20

FIG. 21

% GROWTH

KE 37/1

KE 37/1-III B

AMFI 2 - μg/ml

FIG. 22

% INFECTIOUS CENTRES

AMFI 2 - μg/ml

FIG. 23

36

% GROWTH

KE 37/1

KE 37/1-III B

MHC PEPTIDE - µg/ml

FIG. 24

% INFECTIOUS CENTRES

MHC PEPTIDE - µg/ml

FIG. 25

% GROWTH

KE 37/1

KE 37/1 - III B

DMSO - µg/ml

FIG. 26

% INFECTIOUS CENTRES

DMSO - µg/ml

FIG. 27

% GROWTH

□ KE37/1

▨ KE37/1-III B

HOLST PEPTIDE - µg/ml

FIG. 28

% INFECTIOUS CENTRES

HOLST PEPTIDE - µg/ml

FIG. 29

39

PERCENTAGE P24 PRODUCTION

FIG. 30

PERCENTAGE P25 PRODUCTION, RELATED TO THE CELL NUMBER (OCT. 25, 1990)

FIG. 31

p24 IN CELLS AND SUPERNATANTS AFTER 3h AND 14h OF INCUBATION WITH MELITTIN

FIG. 32

FIG. 33

INFLUENCE OF MELITTIN ON THE p24 DETERMINATION IN
CELL FREE HIV CONTAINING SUPERNATANTS

FIG. 34

FIG. 35

Hymenoptera venoms.  Prolonged toxicity studies - mice.

Body weight grams (g)
Group mean data

| Group | Sex | Body weight (g) at days after onset of dosing | | | | | | | | | | | | |
| | | 0 | 4 | 7 | 12 | 14 | 16 | 47 | 77 | 109 | 138 | 172 | autop. | 200-208 |
|---|---|---|---|---|---|---|---|---|---|---|---|---|---|---|
| Controls | ♂ | 31.4 | 31.4 | 32.0 | 32.2 | 32.6 | 32.2 | 35.0 | 33.6 | 36.2 | 36.4 | 36.2 | 37.0 | |
| " | ♀ | 24.8 | 24.6 | 25.0 | 25.4 | 26.0 | 25.2 | 27.8 | 28.0 | 29.6 | 29.8 | 31.6 | 31.6 | |
| Honey bee | ♂ | 30.4 | 30.4 | 31.0 | 31.8 | 31.4 | 31.2 | 33.2 | 33.2 | 36.0 | 37.4 | 36.4 | 36.2 | |
| "   " | ♀ | 24.4 | 24.2 | 24.8 | 25.0 | 25.4 | 25.0 | 27.6 | 27.8 | 29.0 | 28.4 | 29.8 | 31.0 | |
| Yellow Jacket | ♂ | 31.4 | 31.2 | 31.8 | 32.0 | 32.6 | 32.2 | 34.2 | 35.0 | 35.6 | 36.8 | 35.8 | 36.6 | |
| "     " | ♀ | 23.8 | 23.8 | 24.4 | 24.8 | 25.2 | 25.2 | 26.6 | 28.2 | 29.0 | 29.0 | 30.0 | 30.0 | |
| Hornet Mixture | ♂ | 33.2 | 33.8 | 34.0 | 34.4 | 34.6 | 35.2 | 37.4 | 37.0 | 38.2 | 38.4 | 37.6 | 38.8 | |
| "      " | ♀ | 24.6 | 24.6 | 24.8 | 25.2 | 25.4 | 25.4 | 28.0 | 28.2 | 28.6 | 28.4 | 28.8 | 29.8 | |
| Vespid Mixture | ♂ | 29.4 | 30.2 | 30.8 | 30.8 | 31.2 | 31.4 | 33.0 | 34.2 | 35.2 | 34.4 | 34.8 | 35.0 | |
| "      " | ♀ | 25.0 | 25.2 | 25.4 | 26.2 | 26.0 | 26.2 | 27.6 | 28.4 | 29.8 | 30.2 | 30.4 | 29.4 | |

EP 0 504 191 B1

FIG. 36

Hymenoptera venoms.   Prolonged toxicity studies - mice.

Body weight grams (g)
Individual data

| Mouse No. | Substance | Sex | Body weight (g) at days after onset of dosing | | | | | | | | | | | |
|---|---|---|---|---|---|---|---|---|---|---|---|---|---|---|
| | | | 0 | 4 | 7 | 12 | 14 | 16 | 47 | 77 | 109 | 138 | 172 | (autopsy) 208 |
| 1 | Controls | ♂ | 29 | 29 | 30 | 31 | 31 | 31 | 37 | 33 | 35 | 34 | 34 | 35 |
| 2 | " | " | 31 | 31 | 31 | 31 | 31 | 31 | 32 | 33 | 36 | 37 | 36 | 35 |
| 3 | " | " | 35 | 35 | 36 | 36 | 37 | 36 | 37 | 33 | 35 | 35 | 35 | 39 |
| 4 | " | " | 29 | 29 | 30 | 29 | 29 | 29 | 32 | 34 | 36 | 37 | 37 | 38 |
| 5 | " | " | 33 | 33 | 33 | 34 | 35 | 34 | 37 | 35 | 39 | 39 | 39 | 38 |
| 6 | " | ♀ | 24 | 25 | 25 | 25 | 25 | 24 | 28 | 28 | 29 | 31 | 31 | 33 |
| 7 | " | " | 25 | 24 | 25 | 26 | 26 | 25 | 29 | 28 | 30 | 32 | 35 | 35 |
| 8 | " | " | 25 | 24 | 24 | 25 | 26 | 25 | 27 | 28 | 30 | 29 | 30 | 30 |
| 9 | " | " | 25 | 25 | 25 | 25 | 26 | 25 | 27 | 28 | 30 | 29 | 31 | 30 |
| 10 | " | " | 25 | 25 | 26 | 26 | 27 | 27 | 28 | 28 | 29 | 28 | 31 | 30 |

EP 0 504 191 B1

FIG. 37

Hymenoptera venoms.   Prolonged toxicity studies - mice.

Body weight grams (g)
Individual data

| Mouse No. | Substance | Sex | Body weight (g) at days after onset of dosing | | | | | | | | | | | |
|---|---|---|---|---|---|---|---|---|---|---|---|---|---|---|
| | | | 0 | 4 | 7 | 12 | 14 | 16 | 47 | 77 | 109 | 138 | 172 | (autopsy) 200-4 |
| 101 | Honey bee | ♂ | 29 | 29 | 29 | 30 | 29 | 29 | 31 | 31 | 34 | 34 | 33 | 35 |
| 102 | " | " | 32 | 32 | 32 | 33 | 33 | 33 | 35 | 34 | 40 | 42 | 43 | 37 |
| 103 | " | " | 31 | 31 | 32 | 33 | 32 | 32 | 33 | 33 | 34 | 35 | 32 | 34 |
| 104 | " | " | 30 | 30 | 31 | 31 | 31 | 30 | 32 | 32 | 33 | 35 | 36 | 36 |
| 105 | " | " | 30 | 30 | 31 | 32 | 32 | 32 | 35 | 36 | 39 | 41 | 38 | 39 |
| 106 | " | ♀ | 24 | 23 | 24 | 24 | 25 | 25 | 28 | 26 | 28 | 28 | 28 | 29 |
| 107 | " | " | 24 | 24 | 24 | 25 | 26 | 25 | 27 | 27 | 28 | 28 | 30 | 29 |
| 108 | " | " | 24 | 23 | 25 | 25 | 24 | 24 | 27 | 28 | 28 | 27 | 28 | 31 |
| 109 | " | " | 26 | 27 | 26 | 27 | 27 | 26 | 30 | 32 | 33 | 31 | 33 | 32 |
| 110 | " | " | 24 | 24 | 25 | 24 | 25 | 25 | 26 | 26 | 28 | 28 | 30 | 34 |

EP 0 504 191 B1

FIG. 38

Hymenoptera venoms.  Prolonged toxicity studies - mice.

Body weight grams (g)
Individual data

| Mouse No. | Substance | Sex | Body weight (g) at days after onset of dosing | | | | | | | | | | | |
|---|---|---|---|---|---|---|---|---|---|---|---|---|---|---|
| | | | 0 | 4 | 7 | 12 | 14 | 16 | 47 | 77 | 109 | 138 | 172 | (autopsy) 201-4 |
| 201 | Yellow Jacket | ♂ | 34 | 34 | 35 | 35 | 36 | 35 | 38 | 39 | 40 | 40 | 37 | 38 |
| 202 | " | " | 30 | 30 | 30 | 30 | 31 | 31 | 33 | 35 | 35 | 36 | 36 | 35 |
| 203 | " | " | 32 | 32 | 32 | 33 | 33 | 33 | 33 | 33 | 35 | 37 | 37 | 39 |
| 204 | " | " | 31 | 31 | 32 | 32 | 33 | 33 | 35 | 35 | 34 | 37 | 38 | 37 |
| 205 | " | " | 30 | 29 | 30 | 30 | 30 | 29 | 32 | 33 | 34 | 34 | 31 | 34 |
| 206 | " | ♀ | 23 | 23 | 23 | 24 | 24 | 24 | 24 | 26 | 26 | 26 | 29 | 27 |
| 207 | " | " | 24 | 24 | 25 | 25 | 25 | 25 | 27 | 29 | 30 | 29 | 32 | 32 |
| 208 | " | " | 23 | 24 | 24 | 24 | 25 | 25 | 27 | 29 | 30 | 32 | 30 | 32 |
| 209 | " | " | 25 | 25 | 25 | 25 | 26 | 26 | 27 | 29 | 29 | 28 | 28 | 29 |
| 210 | " | " | 24 | 23 | 25 | 26 | 26 | 26 | 28 | 28 | 30 | 30 | 31 | 30 |

EP 0 504 191 B1

FIG. 39

Hymenoptera venoms.   Prolonged toxicity studies - mice.

Body weight grams (g)
Individual data

| Mouse No. | Substance | Sex | Body weight (g) at days after onset of dosing | | | | | | | | | | | |
|---|---|---|---|---|---|---|---|---|---|---|---|---|---|---|
| | | | 0 | 4 | 7 | 12 | 14 | 16 | 47 | 77 | 109 | 138 | 172 | (autopsy) 202-7 |
| 301 | Hornet mixture | ♂ | 34 | 35 | 35 | 35 | 36 | 36 | 39 | 39 | 42 | 40 | 38 | 40 |
| 302 | " | " | 30 | 31 | 31 | 32 | 32 | 33 | 34 | 34 | 33 | 36 | 36 | 36 |
| 303 | " | " | 34 | 34 | 35 | 35 | 35 | 36 | 36 | 34 | 36 | 35 | 37 | 39 |
| 304 | " | " | 32 | 33 | 33 | 33 | 34 | 34 | 38 | 38 | 38 | 43 | 40 | 39 |
| 305 | " | " | 36 | 36 | 36 | 37 | 36 | 37 | 40 | 40 | 42 | 38 | 37 | 40 |
| 306 | " | ♀ | 25 | 25 | 25 | 25 | 26 | 26 | 28 | 28 | 28 | 28 | 25 | 24 |
| 307 | " | " | 23 | 23 | 23 | 24 | 24 | 23 | 27 | 26 | 26 | 25 | 26 | 29 |
| 308 | " | " | 26 | 25 | 26 | 27 | 26 | 26 | 30 | 32 | 31 | 31 | 34 | 36 |
| 309 | " | " | 23 | 24 | 24 | 24 | 24 | 25 | 27 | 26 | 28 | 28 | 29 | 28 |
| 310 | " | " | 26 | 26 | 26 | 26 | 27 | 27 | 28 | 29 | 30 | 30 | 30 | 32 |

EP 0 504 191 B1

FIG. 40

Hymenoptera venoms.   Prolonged toxicity studies - mice.

Body weight grams (g)
Individual data

| Mouse No. | Substance | Sex | Body weight (g) at days after onset of dosing | | | | | | | | | | | |
|---|---|---|---|---|---|---|---|---|---|---|---|---|---|---|
| | | | 0 | 4 | 7 | 12 | 14 | 16 | 47 | 77 | 109 | 138 | 172 | (autopsy) 202-7 |
| 401 | Vespid Mixture | ♂ | 29 | 30 | 29 | 30 | 30 | 31 | 31 | 32 | 35 | 36 | 36 | 35 |
| 402 | " | " | 27 | 27 | 28 | 28 | 28 | 28 | 30 | 31 | 32 | 32 | 33 | 32 |
| 403 | " | " | 30 | 31 | 32 | 31 | 32 | 31 | 34 | 36 | 37 | 33 | 33 | 34 |
| 404 | " | " | 30 | 31 | 32 | 32 | 33 | 33 | 35 | 37 | 37 | 37 | 38 | 39 |
| 405 | " | " | 31 | 32 | 33 | 33 | 33 | 34 | 35 | 35 | 35 | 34 | 34 | 35 |
| 406 | " | ♀ | 26 | 26 | 26 | 27 | 26 | 27 | 28 | 28 | 30 | 32 | 31 | 29 |
| 407 | " | " | 25 | 25 | 26 | 26 | 26 | 27 | 29 | 30 | 29 | 30 | 30 | 30 |
| 408 | " | " | 23 | 24 | 24 | 25 | 25 | 25 | 25 | 27 | 31 | 29 | 29 | 28 |
| 409 | " | " | 25 | 25 | 25 | 27 | 26 | 26 | 28 | 29 | 29 | 31 | 31 | 31 |
| 410 | " | " | 26 | 26 | 26 | 26 | 27 | 26 | 28 | 28 | 30 | 29 | 31 | 29 |

EP 0 504 191 B1

FIG. 41

Hymenoptera venoms.  Prolonged toxicity studies - mice.
   Microscopic examination.

| Mouse No. | 1 | 2 | 3 | 4 | 5 | 6 | 7 | 8 | 9 | 10 | 101 | 102 | 103 | 104 | 105 | 106 | 107 | 108 | 109 | 110 | 201 | 202 | 203 | 204 | 205 | 206 | 207 | 208 | 209 | 210 |
|---|---|---|---|---|---|---|---|---|---|---|---|---|---|---|---|---|---|---|---|---|---|---|---|---|---|---|---|---|---|---|
| Sex | ♂ ——————→ ♀ ——————————→ | | | | | | | | | | ♂ ——————→ ♀ ——————————→ | | | | | | | | | | ♂ ——————→ ♀ ——————————→ | | | | | | | | | |
| Substance | Controls | | | | | | | | | | Honey Bee | | | | | | | | | | Yellow Jacket | | | | | | | | | |
| Skin | 0 | 0 | 0 | 0 | 0 | 0 | 0 | 0 | 0 | 0 | 0 | 0 | 0 | 0 | - | - | 0 | 0 | 0 | 0 | 0 | 0 | 0 | 0 | 0 | 0 | 0 | 0 | - | 0 |
| Salivary gland | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Trachea | 0 | 0 | - | 0 | 0 | 0 | 0 | 0 | 0 | 0 | 0 | 0 | 0 | - | 0 | 0 | 0 | 0 | 0 | 0 | 0 | 0 | 0 | 0 | 0 | 0 | 0 | 0 | 0 | 0 |
| Lungs and bronchi | 0 | 0 | 0 | 0 | 1 | 0 | 0 | 0 | 0 | 1 | 2 | 1 | 0 | 0 | 2 | - | 0 | 0 | 0 | 0 | 0 | 0 | 0 | 0 | 0 | 1 | 0 | 0 | 0 | 0 |
| Heart and aorta | 0 | 0 | 0 | 0 | 0 | 0 | - | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Thyroids | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | - | 0 | 0 | 0 | 0 |
| Parathyroids | - | 0 | - | - | - | - | - | - | - | 0 | - | - | - | 0 | - | - | 0 | 0 | - | 0 | - | - | - | - | 0 | - | 0 | - | - | - |
| Oesophagus | 0 | 0 | 0 | 0 | 0 | 0 | 0 | - | 0 | - | 0 | 0 | 0 | 0 | 0 | 0 | - | 0 | 0 | 0 | 0 | 0 | 0 | 0 | 0 | 0 | - | 0 | 0 | 0 |
| Stomach | 0 | 0 | 0 | 0 | 0 | 0 | 0 | 0 | 0 | 0 | 0 | 0 | 0 | 0 | 0 | - | 0 | 0 | 0 | - | 0 | 0 | 0 | 0 | 0 | - | 0 | 0 | 0 | 0 |
| Duodenum | 0 | 0 | 0 | 0 | 0 | 0 | 0 | 0 | 0 | 0 | 0 | 0 | 0 | 0 | 0 | | 0 | 0 | 0 | 0 | 0 | 0 | 0 | 0 | 0 | 0 | 0 | - | 0 | 0 |
| Jejunem | 0 | 0 | 0 | 0 | - | 0 | - | 0 | 0 | 0 | 0 | 0 | 0 | 0 | 0 | - | 0 | 0 | 0 | 0 | 0 | 0 | 0 | 0 | 0 | 0 | 0 | 0 | 0 | 0 |
| Ileum | 0 | 0 | 0 | 0 | 0 | 0 | - | 0 | 0 | 0 | 0 | 0 | 0 | 0 | 0 | 0 | - | - | 0 | - | 0 | 0 | 0 | - | 0 | 0 | - | 0 | 0 | 0 |
| Cecum | 0 | 0 | 0 | 0 | 0 | 0 | 0 | 0 | 0 | 0 | 0 | 0 | 0 | 0 | 0 | 0 | 0 | 0 | 0 | 0 | 0 | - | 0 | 0 | 0 | - | 0 | 0 | 0 | 0 |
| Colon | 0 | 0 | 0 | 0 | 0 | 0 | 0 | 0 | 0 | 0 | 0 | 0 | 0 | 0 | 0 | - | 0 | 0 | 0 | 0 | 0 | 0 | 0 | 0 | 0 | 0 | 0 | 0 | 0 | 0 |
| Mesenteric lymph nodes | 0 | 0 | 0 | 0 | 0 | 0 | 0 | 0 | 0 | 0 | 0 | 0 | 0 | 0 | - | 0 | 0 | 0 | 0 | 6 | 0 | 0 | 0 | 0 | 0 | 0 | 0 | 0 | 0 | 7 |
| Liver | 0 | 8 | 0 | 0 | 8 | 8 | 0 | 8 | 0 | 8 | 0 | 8 | 0 | 0 | 0 | 8 | 8 | 0 | 0 | 0 | 0 | 0 | 8 | 0 | 0 | 8 | 0 | 8 | 8 | 8 |
| Gallbladder | 0 | 0 | - | - | 0 | - | 0 | 0 | - | - | - | - | - | - | - | - | 0 | - | 0 | - | 0 | 0 | 0 | - | - | - | 0 | 0 | - | 0 |
| Thigh muscle | 0 | 0 | 0 | 0 | 0 | 0 | 0 | 0 | 0 | 0 | 0 | 0 | - | 0 | 0 | 0 | 0 | 0 | 0 | 0 | 0 | 0 | 0 | 0 | - | 0 | 0 | 0 | 0 | 0 |
| Sciatic nerve | 0 | 0 | - | 0 | 0 | 0 | 0 | 0 | 0 | 0 | 0 | 0 | 0 | 0 | 0 | 0 | 0 | 0 | 0 | 0 | - | 0 | 0 | 0 | - | - | 0 | 0 | - | - |
| Sternebrae | 0 | 0 | 0 | 0 | 0 | 0 | 0 | 0 | 0 | 0 | 0 | - | 0 | 0 | 0 | - | 0 | 0 | 0 | 0 | 0 | 0 | 0 | 0 | 0 | 0 | 0 | 0 | 0 | 0 |
| Thymus | 0 | - | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | - | 0 | 0 |
| Pancreas | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | - | 0 | 0 | 0 | 0 | 0 |
| Spleen | 0 | 0 | 0 | 0 | 0 | 0 | 0 | - | 0 | 0 | 0 | 0 | 0 | 0 | 0 | - | 0 | 0 | 0 | 0 | 0 | 0 | 0 | 0 | 0 | 0 | 0 | 0 | 0 | 0 |
| Kidneys | 0 | 0 | 0 | 0 | 0 | 10 | 0 | 10 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Adrenals | 0 | - | - | 0 | 0 | - | - | 0 | 0 | - | 0 | 0 | 0 | 0 | 0 | 0 | - | - | 0 | 0 | - | - | 0 | 0 | - | 0 | 0 | 0 | 0 | 0 |
| Bladder | 0 | - | 0 | - | 0 | - | 0 | 0 | 0 | 0 | 0 | 0 | - | 0 | 0 | 0 | 0 | 0 | - | 0 | - | 0 | 0 | 0 | - | 0 | 0 | - | 0 | 0 |
| Seminal vesicle | 0 | 0 | 0 | 0 | 0 | | | | | | 0 | 0 | 0 | - | 0 | | | | | | 0 | 0 | 0 | 0 | 0 | | | | | |
| Prostate | 0 | 0 | 0 | 0 | 0 | | | | | | 0 | 0 | 0 | 0 | 0 | | | | | | 0 | - | 0 | 0 | - | | | | | |
| Testes | 0 | 0 | 0 | 0 | 0 | | | | | | 0 | 0 | 0 | 0 | 0 | | | | | | 0 | 0 | 0 | 0 | 0 | | | | | |
| Ovaries | | | | | | 0 | 0 | - | 0 | 0 | | | | | | - | 0 | 0 | 0 | 0 | | | | | | 0 | 0 | 0 | 0 | 0 |
| Uterus | | | | | | - | 0 | 0 | 0 | 0 | | | | | | - | 0 | 0 | 0 | 0 | | | | | | 0 | 0 | 0 | 0 | 0 |
| Brain | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 |
| Pituitary gland | 0 | 0 | - | - | 0 | 0 | 0 | - | 0 | 0 | 0 | 0 | - | 0 | 0 | 0 | 0 | 0 | - | - | 0 | 0 | - | 0 | 0 | - | - | 0 | 0 | - |
| Eyes | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | - |
| Spinal chord | 0 | 0 | 0 | 0 | - | 0 | 0 | 0 | 0 | - | 0 | 0 | 0 | 0 | 0 | 0 | 0 | 0 | 0 | - | 0 | 0 | 0 | 0 | 0 | 0 | 0 | 0 | 0 | 0 |
| Injection sites | 0 | 0 | 0 | 0 | 0 | 0 | 0 | 0 | - | 0 | 0 | 0 | 0 | 0 | 0 | 0 | 0 | 0 | 0 | 0 | 0 | 0 | 0 | 0 | - | 0 | 0 | 0 | 0 | 0 |

Organs with a histologically normal picture are assigned with 0 whereas organs deviating from the normal are given a picture that is explained in the succeeding page.  Organs assigned '-' have not been examined histopathologically.

FIG. 41A

Hymenoptera venoms.  Prolonged toxicity studies - mice.

Legends for Fig. 41.


1.  Focally small accumulations of lymphoid cells.

2.  A small adenoma, probably originating from bronchial
    epithellum.

3.  A small nodular hyperplasia of bronchial epithelium.

4.  A small, oldish and indistinct necrosis with infiltrations
    of polymorphonuclear granulocytes.

5.  Focally oldish necroses with heavy infiltration of
    polymorphs.

6.  Widespread and indistinct oldish necroses with infiltration
    of numerous polymorphs.

7.  A moderately lymphoid hyperplasia.

8.  Necrosis of a few liver cells with slight cellular
    infiltration.

9.  A focal necrosis, slightly demarcated by fibrous tissue and
    strongly infiltrated with polymorphs and macrophages.

10.  A slight chronic pyelonephritis.

11.  A chronic fibrous adhesion to the abdominal wall.

12.  A chronic fibrous and well vascularized adhesion to the
     pancreas.

FIG. 42

## Mellitin analogues and other peptides used:

**1. Mellitin:**

Sequence: Gly-Ile-Gly-Ala-Val-Leu-Lys-Val-Leu-Thr-Thr-Gly-Leu-Pro-Ala-Leu-Ile-Ser-Trp-Ile-Lys-Arg-Lys-Arg-Gln-Gln-Amide.

**2. Mellitin acid:**

Sequence: Gly-Ile-Gly-Ala-Val-Leu-Lys-Val-Leu-Thr-Thr-Gly-Leu-Pro-Ala-Leu-Ile-Ser-Trp-Ile-Lys-Arg-Lys-Arg-Gln-Gln-Acid.

**3. Mellitin 6:**

Sequence: Gly-Ile-Gly-Ala-Val-Leu-Lys-Val-Leu-Thr-Thr-Gly-Leu-Pro-Ala-Leu-Ile-Ser-Trp-Ile-Lys-Lys-Lys-Lys-Gln-Gln-Amide.

**4. Mellitin 4:**

Sequence: Gly-Ile-Gly-Ala-Val-Leu-Lys-Val-Leu-Thr-Thr-Gly-Leu-Pro-Ala-Leu-Ile-Ser-Trp-Ile-Lys-Arg-Lys-Arg-Gly-Gly-Amide.

**5. Mellitin E:**

Sequence: Gly-Ile-Gly-Ala-Val-Leu-Lys-Val-Leu-Thr-Thr-Gly-Leu-Pro-Ala-Leu-Ile-Ser-Trp-Ile-Gly-Gly-Gly-Gly-Gly-Gly-Amide.

**6. Mellitin F:**

Sequence: Gly-Ile-Gly-Ala-Val-Leu-Lys-Val-Leu-Thr-Thr-Gly-Leu-Pro-Ala-Ile-Ser-Trp-Ile-Orn-Orn-Amide.

**7. Mellitin 1-20:**

Sequence: Gly-Ile-Gly-Ala-Val-Leu-Lys-Val-Leu-Thr-Thr-Gly-Leu-Pro-Ala-Ile-Ser-Trp-Ile-Amide.

**8. Amfi 1:**

Sequence: Gly-Thr-Asp-Arg-Val-Ile-Glu-Val-Val-Gln-Gly-Ala-Cys-Arg-Ala-Ile-Arg-His-Ile-Pro-Arg-Arg-Ile-Arg-Gln-Gly-Amide.

**9. Amfi 2:**

Sequence: Gly-Gln-Arg-Val-Arg-Asn-Val-Ile-Ser-Leu-Val-Ala-Phe-Val-Ile-Arg-Leu-Gly-Val-Leu-Gly-Gly-Val-Ile-Met-Ile-Phe-Amide.

**10. MHC:**

Sequence: Val-Ala-Ala-Lys-Ala-Asn-Arg-Val-Ala-Asp-Glu-Ile-Arg-His-Lys-Arg-Glu-Lys-Leu-Glu-Amide.

**11. Holst:**

Sequence: Phe-Ala-Glu-Ser-Gly-Val-Asp-Thr-Pro-Val-Phe-Asn-Ser-Tyr-Amide.